(19) Europäisches Patentamt
European Patent Office
Office européen des brevets

(11) **EP 1 934 368 B1**

(12) **EUROPEAN PATENT SPECIFICATION**

(45) Date of publication and mention
of the grant of the patent:
**26.12.2012 Bulletin 2012/52**

(51) Int Cl.:
*C12Q 1/68* (2006.01)    *G01N 33/66* (2006.01)
*G01N 33/92* (2006.01)

(21) Application number: **06792317.7**

(22) Date of filing: **28.09.2006**

(86) International application number:
**PCT/EP2006/009451**

(87) International publication number:
**WO 2007/039232 (12.04.2007 Gazette 2007/15)**

(54) **MEANS AND METHODS FOR DIAGNOSING ATGL RELATED DISORDERS**

MITTEL UND VERFAHREN ZUR DIAGNOSE VON ERKRANKUNGEN IN VERBINDUNG MIT ATGL

MOYENS ET PROCEDES DE DIAGNOSTIC DE TROUBLES LIES A LIPASE ATGL

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.09.2005 EP 05021217**

(43) Date of publication of application:
**25.06.2008 Bulletin 2008/26**

(73) Proprietor: **Medizinische Universität Innsbruck 6020 Innsbruck (AT)**

(72) Inventors:
- **KRONENBERG, Florian**
  **A-6020 Innsbruck (AT)**
- **HEID, Iris**
  **93055 Regensburg (DE)**
- **SCHOENBORN, Veit**
  **87435 Kempten (DE)**

(74) Representative: **Vossius & Partner Siebertstrasse 4 81675 München (DE)**

(56) References cited:
- DATABASE EMBL [Online] XP002435479 retrieved from EMBL Database accession no. BC017280
- DATABASE EMBL [Online] XP002435480 retrieved from EMBL Database accession no. bc011958
- DATABASE EMBL [Online] XP002435481 retrieved from EMBL Database accession no. ap006621
- DATABASE EMBL [Online] XP002435482 retrieved from EMBL Database accession no. ap006623
- DATABASE EMBL [Online] XP002435483 retrieved from EMBL Database accession no. Q5EFF5
- DATABASE EMBL [Online] XP002435484 retrieved from EMBL Database accession no. aj278476
- ZIMMERMANN R ET AL: "Fat mobilization in adipose tissue is promoted by adipose triglyceride lipase" SCIENCE, AMERICAN ASSOCIATION FOR THE ADVANCEMENT OF SCIENCE,, US, vol. 306, no. 5700, 19 November 2004 (2004-11-19), pages 1383-1386, XP002357247 ISSN: 0036-8075
- LAKE ANDREW C ET AL: "Expression, regulation, and triglyceride hydrolase activity of Adiponutrin family members" JOURNAL OF LIPID RESEARCH, vol. 46, no. 11, 8 September 2005 (2005-09-08), pages 2477-2487, XP002435465 ISSN: 0022-2275
- KRALISCH ET AL: "Isoproterenol, TNFalpha, and insulin downregulate adipose triglyceride lipase in 3T3-L1 adipocytes" MOLECULAR AND CELLULAR ENDOCRINOLOGY, AMSTERDAM, NL, vol. 240, no. 1-2, 30 August 2005 (2005-08-30), pages 43-49, XP005026121 ISSN: 0303-7207
- RABEN D M ET AL: "A new lipase in regulating lipid mobilization: hormone-sensitive lipase is not alone" TRENDS IN ENDOCRINOLOGY AND METABOLISM, ELSEVIER SCIENCE PUBLISHING, NEW YORK, NY, US, vol. 16, no. 2, March 2005 (2005-03), pages 35-36, XP004768091 ISSN: 1043-2760

EP 1 934 368 B1

- JENKINS CHRISTOPHER M ET AL: "Identification, cloning, expression, and purification of three novel human calcium-independent phospholipase A2 family members possessing triacylglycerol lipase and acylglycerol transacylase activities" JOURNAL OF BIOLOGICAL CHEMISTRY, AMERICAN SOCIETY OF BIOLOCHEMICAL BIOLOGISTS, BIRMINGHAM,, US, vol. 279, no. 47, 19 November 2004 (2004-11-19), pages 48968-48975, XP002357248 ISSN: 0021-9258
- VILLENA JOSEP A ET AL: "Desnutrin, an adipocyte gene encoding a novel patatin domain-containing protein, is induced by fasting and glucocorticoids - Ectopic expression of desnutrin increases triglyceride hydrolysis" JOURNAL OF BIOLOGICAL CHEMISTRY, AMERICAN SOCIETY OF BIOLOCHEMICAL BIOLOGISTS, BIRMINGHAM,, US, vol. 279, no. 45, 5 November 2004 (2004-11-05), pages 47066-47075, XP002357249 ISSN: 0021-9258
- SCHOENBORN VEIT ET AL: "The ATGL gene is associated with free fatty acids, triglycerides, and type 2 diabetes" DIABETES, vol. 55, no. 5, May 2006 (2006-05), pages 1270-1275, XP002435466 ISSN: 0012-1797

**Description**

The present invention relates to methods of diagnosing a disease (or a predisposition for a disease) related to the presence of a molecular variant of the adipose triglyceride lipase (ATGL) gene comprising determining in a sample from a subject the presence of at least one nucleic acid comprising a mutation in the 5'UTR, exons, introns or 3'UTR of the ATGL gene. Moreover, the present invention relates to a nucleic acid molecule comprising a nucleotide sequence encoding an adipose triglyceride lipase and comprising one or more of the mutations in the 5'UTR, exons, introns or 3'UTR of the ATGL gene. Furthermore, a diagnostic composition comprising the aforementioned nucleic acid molecule is envisaged by the present invention.

FFA (free fatty acids) are an important energy source in body tissues. In situations of high demand for energy, adipose tissue lipolysis is stimulated, thereby increasing FFA availability. The need for FFA is especially high during starvation or exercise. In addition, FFA have other important physiological functions, for instance, FFA enhance both basal and glucose-stimulated insulin secretion.

Plasma FFA concentrations are elevated in obese individuals, probably caused by an increased FFA release from the expansion in fat mass. Elevated plasma FFA are observed in type 2 diabetes and evidence suggests that early changes may be predictive for the transition of patients from impaired glucose tolerance (IGT) to type 2 diabetes. Studies indicate that elevated circulating FFA may directly contribute to the underlying pathophysiology of type 2 diabetes and the development of insulin resistance. High plasma FFA concentrations are associated with a number of cardiovascular risk factors linked to insulin resistance including hypertension, dyslipidemia, hyperuricemia and abnormal fibrinolysis. Chronically elevated plasma FFA may contribute to β-cell dysfunction in type 2 diabetes (°for review, see reference 1°). The increase of high triglycerides and FFAs results in a lipid overload which has consequences to many organ systems such as heart, skeletal muscle, pancreas, liver and kidney. This lipid overload is also known as °lipotoxicity°.

Understanding the pathogenetic mechanisms, including the genetic components of diseases associated with elevated plasma levels of FFAs is therefore a challenge for research. The knowledge of such mechanisms would furthermore allow the early detection of individuals having a predisposition for such disease which are associated with a changed plasma level of FFAs.

Thus, the technical problem underlying the present invention was to provide means and methods for detecting diseases which are associated with a changed plasma level of FFAs.

The solution to said technical problem is achieved by providing the embodiments characterized in the claims.

As detailed herein below and also summarized in the experimental part of this invention, the present invention provides for a method of predicting/diagnosing a disease or a predisposition for a disease which is related to the presence of the molecular variant of ATGL. As also documented in the appended Figures specific polymorphisms of the ATGL gene as disclosed herein indicate changes in the plasma concentration of free fatty acids. The accumulation of free fatty acids and in particular of excess lipids in non-adipose tissues lead to cell dysfunctions and/or cell death. Accordingly, the higher plasma concentrations of free fatty acids leads to the pathogenesis of, for example, diabetes in particular type II diabetes mellitus as well as to coronary disorders as well as cardiovascular diseases. These disorders comprise heart failures, in particular myocardial infarctation, as well as ischemic heart disease, like angina pectoris. However, high concentrations of free fatty acids may also lead to further diseases, like for example cardiovascular diseases in the brain, in particular to stroke. As also summarized in Schaffer (2003) Current Opinion in Lipidology, 14, 281-287 genes furthermore associated with a lipid or FFA overload may not only affect cells like cardiac myocytes but also hepatocytes and muscle cells. Accordingly, the disease to be predicted in a diagnosis by the methods of the present invention is not limited to the herein disclosed type II diabetes mellitus but may also comprise the diagnosis of heart diseases, liver diseases, diseases of the skeletal muscle, as well as the kidney. Accordingly, the disorders to be diagnosed in accordance with the invention are certainly not limited to type II diabetes mellitus but also comprise disorders like obesity in general as well as cardiophysiological consequences in organs like skeletal muscle, liver, pancreas and kidney. For example in the liver the elevated FFA contributes to disorders like hyperglycaemia, by antagonizing the effects of insulin on endogenous glucose production. With the method of the present invention, i.e. linkage to elevated FFA also the development or the production of a development of a muscle insulin resistance can be predicted and/or diagnosed.

Firstly this disclosure contains a method of diagnosing a disease (or a predisposition for a disease) related to the presence of a molecular variant of the adipose triglyceride lipase (ATGL) comprising determining in a sample the presence of at least one nucleic acid sequence selected from the group consisting of:

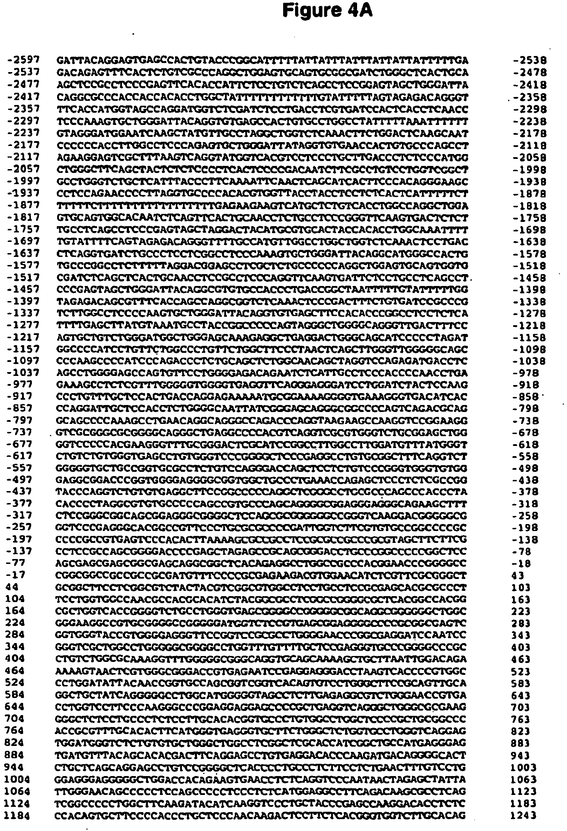

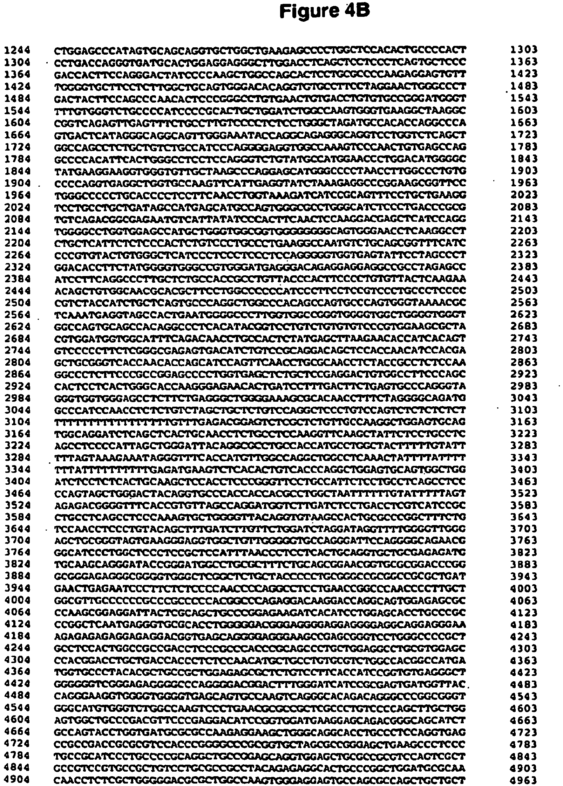

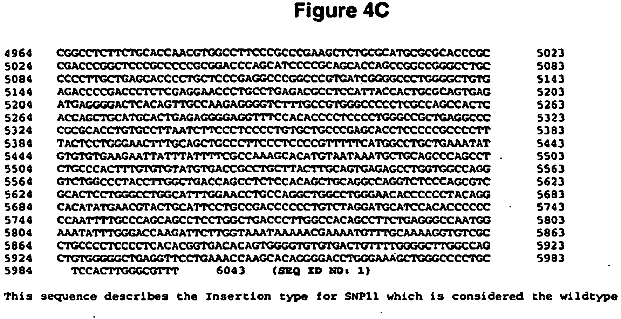

(a) a nucleic acid sequence encoding adipose triglyceride lipase and which contains a mutation in the 5'UTR region corresponding to position -2210 or -2014 of the genomic nucleotide sequence of the wild-type adipose triglyceride lipase as depicted in SEQ ID NO: 1 (Figures 4A to C) wherein at said position a nucleotide is replaced by a different nucleotide;

(b) a nucleic acid sequence encoding adipose triglyceride lipase, wherein in the exon as indicated in column "Exon" of the following Table A the nucleotide as indicated in column "Replaced nucleotide" of Table A corresponding to the position as indicated in column "Position in wild-type" of Table A of the nucleotide sequence of the wild-type adipose triglyceride lipase as depicted in SEQ ID NO: 1 (Figures 4A to C) is replaced by a different nucleotide

**Table A**

| Exon | Replaced nucleotide | Position in wild-type |
|---|---|---|
| exon 5 | C | 2847 |
| exon 6 | C | 3871 |
| exon 7 | C | 4091 |
| exon 10 | C | 5071 |

(c) a nucleic acid sequence encoding adipose triglyceride lipase, wherein nucleotides corresponding to positions 5274 to 5277 of the genomic nucleotide sequence of the wild-type adipose triglyceride lipase as depicted in SEQ ID NO: 1 (Figures 4A to C) are deleted;

(d) a nucleic acid sequence encoding adipose triglyceride lipase, wherein at least one nucleotide is added starting at position 5274 of the genomic nucleotide sequence of the wild-type adipose triglyceride lipase as depicted in SEQ ID NO: 1 (Figures 4A to C);

(e) a nucleic acid sequence encoding adipose triglyceride lipase, wherein in the intron as indicated in column "Intron" of the following Table B, the nucleotide as indicated in column "Replaced nucleotide" of Table B corresponding to the position as indicated in column "Position in wild-type" of Table B of the genomic nucleotide sequence of the wild-type adipose triglyceride lipase as depicted in SEQ ID NO: 1 (Figures 4A to C) is replaced by a different nucleotide

**Table B**

| Intron | Replaced nucleotide | Position in wild-type |
|---|---|---|
| intron 2 | C | 550 |
| intron 5 | T | 2904 |

(f) a nucleic acid sequence encoding adipose triglyceride lipase which contains a mutation in the 3'UTR region corresponding to position 5392 of the genomic nucleotide sequence of the wild-type adipose triglyceride lipase as depicted in SEQ ID NO: 1 (Figures 4A to C), wherein at said position a nucleotide is replaced by a different nucleotide;

(g) a nucleic acid sequence comprising at least 15 nucleotides of the nucleic acid sequence of any one of (a) to (f) and comprising one or more of the mutations as defined in any one of (a) to (f);

(h) a nucleic acid sequence comprising a nucleotide sequence as shown in SEQ ID No: 11;

(i) a nucleic acid sequence encoding a polypeptide comprising the amino acid sequence of SEQ ID NOs: 7, 10 or 13;

(j) a nucleic acid sequence which hybridizes to a nucleotide sequence defined in any one of (a) to (i) and having a mutation as defined in any one of (a) to (f); and

(k) a nucleic acid sequence being degenerate as a result of the genetic code to the nucleic acid sequence as defined in (j).

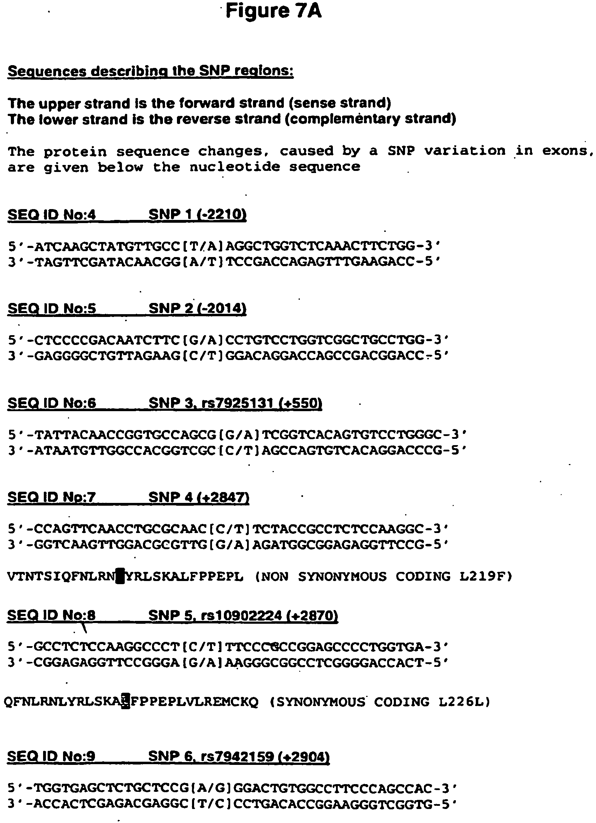

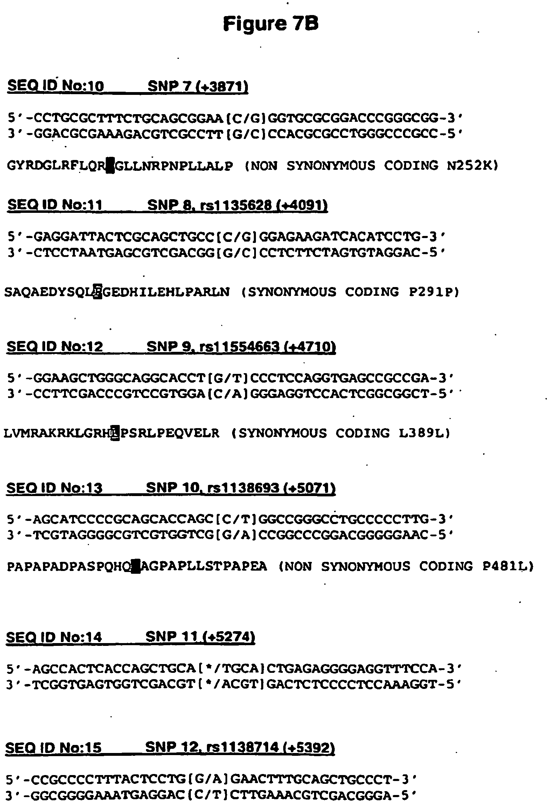

Specific nucleic acid sequences as defined in section (g) above, i.e. nucleic acid sequences comprising at least 15 nucleotides of the nucleic acid sequence of any one of (a) to (f) and comprising one or more of the mutations as defined in any one of (a) to (f) are depicted in Figures 7A and 7B (particularly SEQ ID Nos: 4 to 7, 9 to 11 and 13 to 15).

In accordance with the present invention by the term "sample" is intended any biological sample obtained from an individual, cell line, tissue culture, or other source containing polynucleotides or polypeptides or portions thereof. As indicated, biological samples include body fluids (such as blood, sera, plasma, urine, synovial fluid and spinal fluid) and tissue sources found to express the polynucleotides of the present invention. Methods for obtaining tissue biopsies and body fluids from mammals are well known in the art. A biological sample which includes genomic DNA, mRNA or proteins is preferred as a source.

Decreased FFAs might point to a genetic constellation which protects against disease such as diabetes mellitus, insulin resistance and metabolic syndrome and the diseases associated with that metabolic pathways. Therefore, a decreased FFA level as well as genetic variants within the ATGL gene which are associated with decreased FFA levels might be a marker for a protection against those diseases.

In a method as described above the disease to be diagnosed is associated with an decrease of the free fatty acids.

Understanding of pathogenetic mechanisms, including the genetic components of type 2 diabetes mellitus (T2DM), is a major challenge for research. However, finding genes for a complex disease such as T2DM is an extremely difficult and long-lasting process. One of the reasons is the difficulty in phenotyping disease endpoints, to which many pathways contribute with completely different genes involved. However, for precisely measurable intermediate phenotypes in the disease pathway, the contribution of a single gene can be substantial and studying their association with the gene might be more fruitful than concentrating on phenotypes too far away from the gene product. Plasma level of free fatty acids (FFAs) is such an intermediate phenotype in the pathway of T2DM.

FFAs play a major role in the pathogenesis of T2DM which is underlined by a strong link between increased plasma FFAs and insulin resistance [1]. Serum triglycerides and FFAs are furthermore elevated in type 1 diabetes mellitus and in obese subjects. Furthermore in congenital or required lipodystrophies.

FFAs are released from lipid storages in fasting or energy-demanding states by lipolytic enzymes. It was believed until recently that the hormone-sensitive lipase (HSL) is the primarily responsible enzyme for the hydrolysis of triacylglycerols and diacylglycerols which releases FFAs [2]. Recently, our research consortium and two other groups independently identified a new lipase called adipose triglyceride lipase (ATGL, alternative names: desnutrin, TTS2.2, iPLA2ξ, PNPLA2) [3-5]. Overexpression of *ATGL* enhanced the lipolysis in adipocytes and inhibition did the opposite [3]. Villena and colleagues observed the level of mRNA highly upregulated in fasting mice and reduced after refeeding [5]. This lipase was demonstrated to catalyze the initial step of the breakdown of triacylglycerol molecules with high substrate specificity by selectively hydrolyzing the primary ester bond in adipose tissue of mammals [3]. The hydrolyzing of the second ester bond is the domain of HSL. Therefore, both enzymes might substantially contribute to the pool of FFAs [2].

We resequenced the entire *ATGL* gene in 48 normal-weight and 48 obese subjects (see the appended Examples including the appended Tables). From the SNPs detected we selected 11 SNPs for the following reasons ordered by decreasing priority: resulting amino acid exchange, location at the intron-exon boundary, location in the untranslated 3'UTR region, minor allele frequency above 5%, or being a tagging SNP. In addition, we selected one 4 basepair insertion/ deletion polymorphism at position +5274. These 12 polymorphisms (Table 2) were genotyped in a large group of 2434 Caucasian individuals from Utah, USA, recruited either for severe obesity (BMI between 35 and 90 kg/m², n=1101), coronary artery disease (n=469) or as randomly selected controls from the same ethnicity (n=864) **(Supplementary Table D** of the appended Examples). None of the genotyped SNPs violated Hardy-Weinberg equilibrium. Four SNPs were rather rare with a minor allele frequency below 5%. One further variant (SNP4) was a very rare mutation (n=1 with minor allele) and thus discarded from analysis. The genotype and allele frequencies did not differ between the three subgroups.

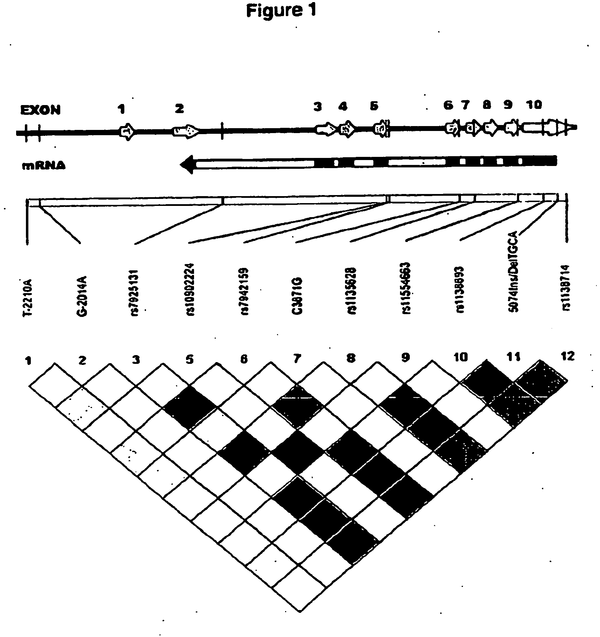

The squared correlation coefficient ($r^2$) as well as gene structure of *ATGL* are depicted in Figure 1. There were three SNPs without notable correlation to any other (SNP1, 2, and 7 ; r<0.5). The rare polymorphisms 5 and 9 were highly correlated (r=0.95). SNP3, 6, 8, 10, 11, and 12 showed high interSNP correlations (r>0.6) with SNP6 and 12 (r=0.94) and SNP3, 8, 10, 11 ($r^2$>= 0.9) being particularly close. Lewontin's D' was high throughout the gene with D' above 0.9 for all pairs of consecutive SNPs which points to a single block structure of the gene.

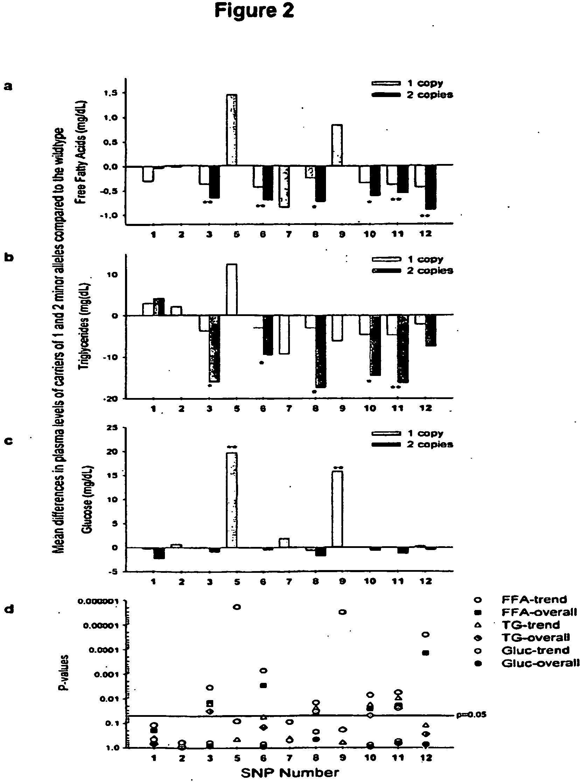

Figure 2a summarizes the results of our primary analysis of the SNP association with FFA concentrations using linear regression analysis. All parameters were tested on the log-scale to assure normally distributed residuals. It can be seen that the two correlated SNPs, SNP6 and 12, as well as SNP3, 8, 10, 11 are significantly associated with decreased FFA levels showing a clear trend per copy of the minor allele (p-values between 0.015 and 0.00003 for an additive inheritance model). Considering that the mean plasma value of FFAs is 7.7 mg/dL (standard deviation 3.6 mg/dL), the changes in mean levels were approximately 15-20% of the mean, ranging from -1 mg/dL to +1.5 mg/dL. Computing the number of effective loci as 9.1 by the Nyholt approach [6] yielded a significance level of 0.006, which can be assumed to conservatively account for the multiple testing of the 11 SNPs.

The pattern was the same when we analyzed triglyceride concentrations in our secondary analysis (Fig. 2b) with p-values less pronounced compared to FFA levels (lowest p-value=0.01 for SNP11). A weaker association of *ATGL* with triglycerides than with FFA levels is in line with the physiological observation that only a limited amount of FFAs is oxidized in the liver and the majority is reesterified to triglycerides which are again transported to adipose tissue for storage [7]. In addition to triglycerides being derived from nutritional intake, this process contributes to the circulating triglyceride plasma concentrations. Triglycerides thus appear to have a larger distance from the *ATGL* gene than FFAs in that pathway.

Causal SNPs leading to such results are difficult to pinpoint on the basis of genetic association studies in general. However, the non-synonymous exchange in SNP10 (Exon 10) or the insertion/deletion polymorphism in the 3'UTR region could be causally involved as, for example, the variations in the 3'UTR region could influence the mRNA stability. The present invention as described herein thus discloses for the first time SNPs of the ATGL-gene which are associated with an decrease in the level of free fatty acids (FFAs) in the plasma of a subject, preferably a human being.

In contrast to the other SNPs, SNP5 and SNP9 were associated with higher rather than lower FFA and triglyceride levels. Furthermore, there was a strong association with an increase in fasting glucose concentrations of 20 and 16 mg/dL, respectively, in carriers of the minor allele (frequency 0.6 and 0.9%, respectively) compared to the wildtype (p<0.00001 for both SNPs) (Fig. 2c). None of the other SNPs revealed an association with glucose levels. Consistently, these two SNPs showed an association with an increased risk for T2DM with an OR of 2.65 (95%CI 1.14-6.14, p=0.02). Though the number of minor allele carriers was rather small per subgroup, the increased OR was found in all three subgroups. The T2DM risk estimate was not altered by additionally adjusting for body mass. In the light of the various pathways leading to T2DM and the numerous genes involved in these pathways, it is noteworthy that these two *ATGL* variants affecting 0.5% of the studied population and thus accounting for almost 1% of T2DM cases (population attributable risk) seem to play such a large role. Our findings are in line with observations that FFAs induce hepatic insulin resistance and increase gluconeogenesis which results in an increased hepatic glucose production in case of a defective hepatic autoregulation as seen in diabetogenic conditions [8].

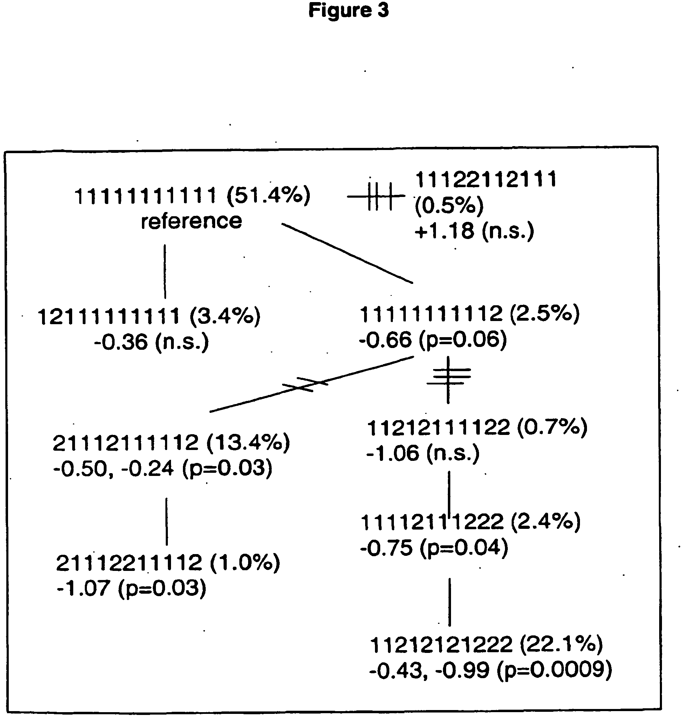

Haplotype analysis provided consistent findings. We statistically reconstructed haplotypes based on the 2228 subjects with all 11 SNPs successfully typed. We identified three common haplotypes (51 %, 22% and 13%) and six rarer haplotypes (0.5% - 5%) which together constitute almost the whole spectrum of haplotype diversity (97.2%) in the *ATGL* gene (Table 1). Haplotype reconstruction error was negligible as the percentage of unambiguously defined haplotypes was 99%. We tested the change of FFA levels (on the log-scale) for subjects with one or two copies of a haplotype against the reference haplotype (most common haplotype). Subjects with at least one haplotype other than reference showed significantly lower FFA levels (7.25, 6.90, 6.30 mg/dL, p<0.0001 with trend for increasing number of haplotypes different from the reference). The results of testing FFA levels for subjects with a certain haplotype against the subjects carrying two copies of reference are summarized in Table 1. It can be seen that the haplotype H6 containing the minor allele of SNP5 and SNP9 is the only haplotype being associated with increased levels of FFAs, whereas all others are associated with decreased levels, when compared to the reference. The statistical significance for 4 of the 9 haplotypes indicates a good differentiation of FFA levels by the *ATGL* haplotypes. The relationship between haplotypes can be viewed in a minimal spanning net (minimal distance from common haplotype in terms of number of mutations, Fig.3). This might give some notion of a genealogy of the haplotypes. Interestingly, the one haplotype being associated with increased FFA levels is located on a separate branch of this net and, being rather rare, might have developed from the most common haplotype later in the population history. Considering the associations of the rare alleles in SNP5 and 9 with blood glucose levels, we observed a pronounced increase of blood glucose levels of 14 mg/dL (p<0.0001) for carriers of haplotype H6.

It is important to state that our overall finding is supported by the fact that the estimates for all of our analyses were consistent in the three subgroups. The associations for FFA, triglycerides and glucose levels as well as the T2DM risk estimate of the SNPs described above pointed towards the same direction. Due to the lower number of individuals per subgroup the p-values in several SNPs no longer reached significance. It is further of importance that the reported estimates were highly robust towards sensitivity analysis: exclusion of one of the subgroups, additionally adjusting for sample origin (GB, CHD, controls), or additionally adjusting for a body mass measure such as BMI or waist circumference did not alter the results. There was no interaction of the described genotype effect with sex nor with the three recruited groups.

Additionally, our findings are further supported by the fact that different phenotypes within the same pathway - FFA, triglycerides, and glucose levels - showed associations in the same direction despite a rather low correlation between each other (e.g. Spearman correlation coefficient between FFAs and triglycerides =0.29, between FFAs and glucose 0.17).

Thus, the present invention relates to a method of diagnosing a disease (or a predisposition for a disease) related to the presence of a molecular variant of the adipose triglyceride lipase (ATGL) gene comprising determining in a sample the presence of at least one nucleic acid sequence selected from the group consisting of:

(a) a nucleic acid sequence encoding adipose triglyceride lipase, wherein in the exon as indicated in column "Exon" of the following Table A the nucleotide as indicated in column "Replaced nucleotide" of Table A corresponding to the position as indicated in column "Position in wild-type" of Table A of the nucleotide sequence of the wild-type adipose triglyceride lipase as depicted in SEQ ID NO: 1 (Figures 4A to C) is replaced by a different nucleotide

**Table C**

| Exon | Replaced nucleotide | Position in wild-type |
|---|---|---|
| exon 5 | C | 2870 |
| exon 9 | G | 4710 |

(b) a nucleic acid sequence comprising at least 15 nucleotides of the nucleic acid sequence of (a) and comprising one or more of the mutations as defined in (a);
(c) a nucleic acid sequence comprising a nucleotide sequence as shown in any one of SEQ ID NOs: 8 and 12;
(d) a nucleic acid sequence which hybridizes to a nucleotide sequence defined in any one of (a) to (c) and having a mutation as defined in (a); and
(e) a nucleic acid sequence being degenerate as a result of the genetic code to the nucleic acid sequence as defined in (d).
Specific nucleic acid sequences as defined in section (b) above, i.e. nucleic acid sequences comprising at least 15 nucleotides of the nucleic acid sequence of (a) and comprising one or more of the mutations as defined in (a) are depicted in Figures 7A and 7B (particularly SEQ ID Nos: 8 and 12).

Preferably, the disease to be diagnosed with the aforementioned method is associated with an increase of the free fatty acids. More preferably, said disease is type 2 diabetes mellitus (T2DM), type 1 diabetes mellitus, metabolic syndrome, insulin resistance, obesity, congenital or required lipodystrophies and related components of those diseases such as hypertension, dyslipidemia and hyperuricaemia.

In summary, our study is the first describing polymorphisms of the *ATGL* gene in humans and a clear association on closely related phenotypes such as free fatty acids and triglyceride concentrations. We even observed an association of a rare mutation of the *ATGL* gene with T2DM. These results complement previous findings of functional studies in mammals and elucidate a role of the *ATGL* gene in pathways involved in components of the metabolic syndrome underscoring the attractive value of *ATGL* as new drug target. Thus, the present invention also contemplates a screening assay which allows the convenient identification and/or isolation of compounds that counteract the increase of free fatty acids in plasma mediated by ATGL such that a normal plasma level of FFAs is restored or essentially restored. Thus, the present invention opens up the possibility to screen for compounds which reduce the production of excess FFAs as mediated by ATGL in a cell/tissue/subject and, thereby, are suitable for treating or preventing a disease which is associated with an excess plasma level of FFAs. These methods can be applied to screen for efficient drugs for the treatment/ prevention of a disease which is associated with the presence of molecular variant of ATGL such as T2DM as described herein above.

In one embodiment, the present invention relates to a method for screening a compound which is suitable for the treatment of a disease such as T2DM which is associated with the presence of a molecular variant of ATGL, said method comprising:

(a) contacting a cell comprising a nucleic acid encoding ATGL which comprises SNP5 and/or SNP9 as defined herein with a test compound;
(b) measuring the effect of the test compound on the lipase activity of said ATGL; and
(c) identifying (a) test compound(s) which reduce the lipase activity of said ATGL. Test-systems which can be employed in this regard are well-known to the skilled person, e.g. from Zimmermann,R. et al. Science 306, 1383-1386 (2004) or Jenkins,C.M. et al. J. Biol. Chem. 279, 48968-48975 (2004), or Methods Mol Biol. 1999;109:109-21, but not limited thereto.

The term "reduced" or "reducing" as used herein defines the reduction of the lipase activity of the ATGL, preferably to at least about the same level as compared to a normal/natural state of a comparable control-cell/subject. Accordingly, it will be understood that the reduction mediated by the test compound which acts as an inhibitor of the ATGL aims at "normalizing" the lipase activity of ATGL, which does, however, not exclude that the inhibitor as defined herein might also reduce the lipase activity of ATGL to a lower level as compared to a normal/natural state of a comparable control-cell/subject. It is also envisaged that the inhibitor totally abolishes the lipase activity of ATGL when compared to a normal/natural state of a comparable control-cell/subject. The term "normal/natural state of a comparable control-cell/ subject" means the lipase activity mediated by ATGL in a control-cell which is preferably of the same nature as the test-cell (e.g. both cell are adipocytes) but which is derived from a different source. "A different source" includes e.g. a cell/ tissue sample obtained from a healthy subject which does not suffer from a disease which is associated with the presence of a molecular variant of ATGL such as T2DM (or other diseases described herein). Assays to classify a disease which is associated with the presence of a molecular variant of ATGL and mediated by an excess lipase activity of ATGL are outlined in the appended examples. However, even in cases where the inhibitor will not reduce the lipase activity of ATGL to the normal/natural state of a comparable control-cell/subject but actually reduces said lipase activity when compared to the ATGL-mediated lipase-activity before the addition of said test-compound(inhibitor), it will be appreciated that said inhibitor has a beneficial effect on the cell/tissue/subject in question. For medical treatment it is preferable to use inhibitors that act in a reversible manner and do not block biochemical processes completely, because such drugs can be applied in a dosage that complies with the desired effect.

Accordingly, it is envisaged that the inhibitor of the invention at least reduces the lipase activity as mediated by a molecular variant of ATGL as described herein to 5%, 10%, 20%, 30%, 40%, 50%, 60%, 70%, 80%, 90% or even 100% when compared to the ATGL-mediated lipase activity that is achieved without the addition of said inhibitor. One specific screening assay for the lipase activity is based on the screening assay disclosed in Zimmermann,R. et al. Science 306, 1383-1386 (2004), but not limited thereto.

The reduction will also depend on the dosage and on the way of administration of the inhibitor. The dosage regimen utilizing the inhibitor of the present invention is therefore selected in accordance with a variety of factors including type, species, age, weight, sex and medical condition of the patient; the severity of the condition to be treated; the route of administration; and the particular compound employed. It will be acknowledged that an ordinarily skilled physician or veterinarian can easily determine and prescribe the effective amount of the compound required to prevent, counter or arrest the progress of the condition. Test-systems which are suitable for such purposes, i.e. which allow to measure the effect of an inhibitor on the ATGL-mediated lipase activity are described herein.

The interaction of the inhibitor with the herein described ATGL such that the lipase activity is reduced, can, in accordance with this invention e.g. be effected by a reduction of the amount of said ATGL in cells, in tissues comprising said cells or subjects comprising said tissues or cells for example by aptamers, antisense oligonucleotides, iRNA or siRNA which specifically bind to the nucleotides sequences encoding said ATGL or by ribozmes which specifically degrade polynucleotides which encode ATGL; by blocking the binding site of the ATGL for lipids; by competitive or allosteric inhibition of the ATGL-mediated lipase activity or by otherwise reducing or preventing the lipase activity of the ATGL described herein, for example by directing antibodies and/or aptamers to ATGL as defined herein and thereby reducing or preventing the lipase activity mediated by said ATGL. Thus, an example of an inhibitor of this invention is an antibody, preferably an antibody the binding of which interferes with the lipase activity of ATGL; an antisense construct, iRNA, siRNA or ribozyme constructs directed against a transcript or the coding nucleotide sequence of the ATGL; nucleotide sequences encoding such constructs and compounds which inhibit the lipase activity as defined herein, for example by blocking or disrupting the binding site of the ATGL for lipids or by allosteric or competitive inhibition of the lipase activity mediated by the ATGL defined herein. The term °test-compound° which is interchangeable with "inhibitor" in accordance with the screening methods of the present invention shall mean any biologically active substance that has an effect on the lipase activity as mediated by ATGL. Preferred compounds are nucleic acids, preferably coding for a peptide, polypeptide, antisense RNA, iRNA, siRNA or a ribozyme or nucleic acids that act independently of their transcription respective their translation as for example an antisense RNA or ribozyme; natural or synthetic peptides, preferably with a relative molecular mass of about 1.000, especially of about 500, peptide analogs polypeptides or compositions of polypeptides, proteins, protein complexes, fusion proteins, preferably antibodies, especially murine, human or humanized antibodies, single chain antibodies, $F_{ab}$ fragments or any other antigen binding portion or derivative of an antibody, including modifications of such molecules as for example glycosylation, acetylation, phosphorylation, farnesylation, hydroxylation, methylation or esterification, hormones, organic or inorganic molecules or compositions, preferably small molecules with a relative molecular mass of about 1.000, especially of about 500.

Test-compounds tested positive for being capable of reducing the lipase activity of the ATGL as described herein are prime candidates for the direct use as a medicament or as lead compounds for the development of a medicament. Naturally, the toxicity of the compound identified and other well-known factors crucial for the applicability of the compound as a medicament will have to be tested.

It is envisaged that the "cell" employed in the screening assays of the present invention is a bacterial, an insect, a fungal, or an animal cell.

The term °bacterial" is meant to include all bacteria which can be transformed or transfected with a DNA or RNA molecules for the expression of ATGL as described herein. Bacterial hosts may include gram negative as well as gram positive bacteria such as, for example, *E. coli, S. typhimurium, Serratia marcescens* and *Bacillus subtilis.* A polynucleotide sequence encoding ATGL can be used to transform or transfect the cell using any of the techniques commonly known to those of ordinary skill in the art. Furthermore, methods for preparing fused, operably linked genes and expressing them in, e.g., mammalian cells and bacteria are well-known in the art (Sambrook et al., "Molecular Cloning: A Laboratory Manual", 3rd edition, Cold Spring Harbor Laboratory, 2001.).

In one embodiment of the screening-methods of the invention, said cell is a mammalian cell or a mammalian cell line.

In a preferred embodiment said mammalian cell or mammalian cell line is derived from human, horse, swine, goat, cattle, mouse or rat.

In a further preferred embodiment of the methods of the invention the cell or cell line is a adipocyte, hepatocyte or a kidney cell or cell line. Such cells or cell lines are well-known to the skilled person.

In a further embodiment of the methods of the invention, said cell is comprised in a tissue, e.g. in adipose tissue, hepatic tissue, kidney tissue.

In a preferred embodiment of the methods of the present invention said cell or said tissue is derived from a mammalian subject preferably a human subject which suffers from a disease which is associated with the presence of a molecular variant of ATGL such as T2DM.

In the context of the present invention the term "subject" means an individual in need of a treatment of an affective disorder. Preferably, the subject is a mammalian, particularly preferred a human, a horse, a camel, a dog, a cat, a pig, a cow, a goat or a fowl.

It will also be understood that further mutations might occur within the ATGL-gene, which as such correlate with the presence of the mutations described herein. Particularly, it is envisaged that e.g. future SNPs are detected within the ATGL-gene which are also correlated with the presence of the SNPs as described herein (particularly, the presence of SNP5 and/or SNP9).

Accordingly, the present invention provides a method for determining whether a (further) mutation in the ATGL gene is associated with the occurrence of at least one of the mutations as defined herein. Preferably, the mutation is SNP5 and/or SNP9 as defined herein. Such correlated SNPs could thus be detected alternatively with the methods of the present invention as described herein above and/or herein below.

In a preferred embodiment the methods described herein comprise PCR, MALDI-TOF, ligase chain reaction, NASBA, restriction digestion, direct sequencing, nucleic acid amplification techniques, hybridization techniques or immunoassays. In accordance with this embodiment of the present invention, the diagnosis of an affective disorder can, e.g., be effected by isolating cells from an individual, and isolating the genomic DNA of said cells. Such cells can be collected from body fluids, skin, hair, biopsies and other sources. Collection and analysis of cells from bodily fluids such as blood, urine and cerebrospinal fluid is well known to the art; see for example, Rodak, "Haematology: Clinical Principles & Applications" second ed., WB Saunders Co, 2002; Brunzel, "Fundamentals of Urine and Body Fluids Analysis", WB Saunders Co, 1994; Herndon and Brumback (Ed.), "Cerebrospinal Fluid", Kluwer Academic Pub., 1989. In addition, methods for DNA isolation are well described in the art; see, for example, Sambrook et al., "Molecular Cloning: A Laboratory Manual", 3rd edition, Cold Spring Harbor Laboratory, 2001.

Once DNA has been isolated, various oligonucleotide primers spanning the SNPs of ATGL as described herein locus may be designed in order to amplify the genetic material by Polymerase Chain Reaction (PCR). Conventional methods for designing, synthesizing, producing said oligonucleotide primers and performing PCR amplification may be found in standard textbooks, see, for example Agrawal (Ed.), "Protocols for Oligonucleotides and Analogs: Synthesis and Properties (Methods in Molecular Biology, 20)", Humana Press, 1993; Innis et al. (Ed.), "PCR Applications: Protocols for Functional Genomics", Academic Press, 1999; Chen and Janes (Ed.), "PCR Cloning Protocols: From Molecular Cloning to Genetic", 2nd edition, Humana Press, 2002. Primers for the detection of ATGL variations are also given in the appended examples. Once DNA has been amplified, nucleotide structure can be analysed by sequencing methods and compared to normal ATGL DNA. Sequencing may be performed manually by any molecular biologist of ordinary skills or by an automated sequencing apparatus. These procedures are common in the art, see, for example, Adams et al. (Ed.), "Automated DNA Sequencing and Analysis", Academic Press, 1994; Alphey, "DNA Sequencing: From Experimental Methods to Bioinformatics", Springer Verlag Publishing, 1997.

Detection and analysis of variations in ATGL encoding nucleic acid sequences can also be performed using amplification refractory mutation system (ARMSTM), amplification refractory mutation system linear extension (ALEXTM), single-strand conformation polymorphism (SSCP), heteroduplex analysis, PCR-SSCP, fluorescent SSCP in an automated DNA sequencer, denaturing gradient gel electrophoresis, RNase protection assays, detection of mutations by sequence specific oligonucleotide hybridization, chemical cleavage methods, enzyme mismatch cleavage methods, cleavage fragment length methods, allele-specific oligonucleotide hybridization on DNA chips or wafers or arrays, and other such methods known in the art, see, for example Nollau et al, Clin. Chem. 43 (1997), 1114-1128; Burczak and Mardis (Ed.), "Polymorphism Detection & Analysis Techniques", Eaton Pub Co, 2000; Cotton et al. (Ed.), "Mutation Detection: A Practical Approach", Irl Press, 1998; Taylor (Ed.), "Laboratory Methods for the Detection of Mutations and Polymorphisms in DNA", CRC Press, 1997.

In accordance with the present invention, the term "nucleic acid sequence" means the sequence of bases comprising purine- and pyrimidine bases which are comprised by nucleic acid molecules, whereby said bases represent the primary structure of a nucleic acid molecule. Nucleic acid sequences include DNA, cDNA (e.g. obtained from spliced and/or unspliced mRNA), genomic DNA, RNA, synthetic forms and mixed polymers, both sense and antisense strands, or may contain non-natural or derivatized nucleotide bases, as will be readily appreciated by those skilled in the art.

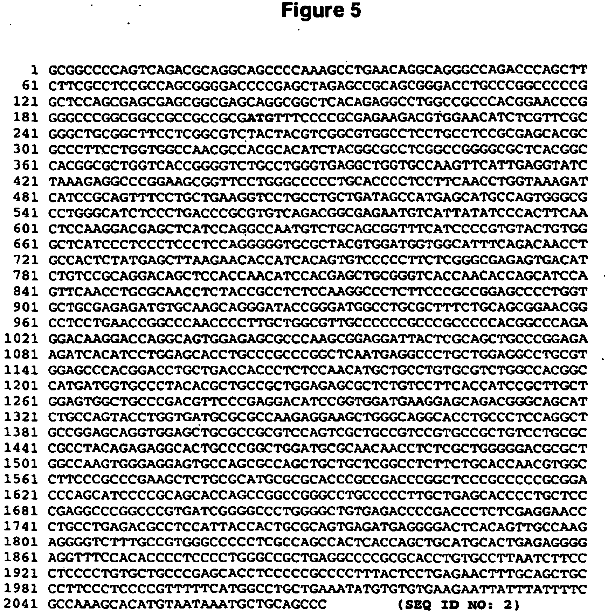

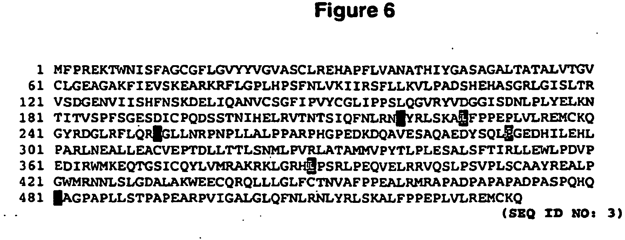

In the context of the present invention the term "molecular variant" ATGL gene or protein as used herein means that said ATGL gene or protein differs from the wild type ATGL gene or protein by at least one nucleotide or amino acid substitution, deletion and/or addition. The genomic sequences of the wildtype ATGL gene as used herein is shown in SEQ ID NO: 1 (Figures 4A to C), the ATGL cDNA sequence is shown in SEQ ID NO: 2 (Figure 5) and the ATGL amino acid sequence is shown in SEQ ID NO: 3 (Figure 6). It will be understood that the methods of the present invention include that instead of determining the presence of a nucleotide sequence having one of the mutations described herein it is, alternatively, also possible to detect the presence of the -encoded variant polypeptide of the described ATGL variants. Particularly, instead of detecting the presence of a nucleic acid sequence comprising any one of SNP4, 7 and 10, it is also possible to detect the presence of the respective protein variant. Means and methods for detecting such polypeptide variants are well-known to the skilled person and/or further described herein. A nucleotide sequence of mouse ATGL is disclosed in NCBI nucleotide entry AK031609 (gi: 26327464). The 1.460 bp coding sequence specifies a putative protein of 486 amino acids (NCBI accession number BAC27476) with a calculated molecular weight of 53.652 D. A nucleotide sequence of human ATGL is disclosed in NCBI nucleotide entry NCBI AK031609 (gi: 26327464; human). However, in the context of the present invention, the sequence as depicted in SEQ ID NO:1 (Figures 4A to C) is referred to as the "wildtype" sequence.

When used herein, the term "polypeptide" means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The terms polypeptide and protein are often used interchangeably herein. The term polypeptide also refers to, and does not exclude, modifications of the polypeptide, e.g., glycosylation, acetylation, phosphorylation and the like. Such modifications are well described in basic texts and in more detailed monographs, as well as in a voluminous research literature.

When used in the context of the present invention the term "a predisposition for a disease" includes a tendency or susceptibility to develop a certain disease that can be triggered under certain conditions, e.g. influence of the environment, personal circumstances of a subject, such as the nutritional behavior of a subject. The tendency or susceptibility to develop a certain disease is associated with a genotype that increases the risk for developing a disease, if other certain conditions, such as those mentioned above, are present. Genetic testing is able to identify subjects who are genetically predisposed to certain health problems. The disease for which a subject has a tendency or susceptibility to develop is preferably a disease related to the presence of a molecular variant of ATGL as described herein.

The term "position" when used in accordance with the present invention means the position of either an amino acid within an amino acid sequence depicted herein or the position of a nucleotide within a nucleic acid sequence depicted herein. The term "corresponding" as used herein also includes that a position is not only determined by the number of the preceding nucleotides/amino acids. The position of a given nucleotide in accordance with the present invention which may be deleted, substituted or comprise one or more additional nucleotide(s) may vary due to deletions or additional nucleotides elsewhere in the ATGL promoter or gene. Thus, under a "corresponding position" in accordance with the present invention it is to be understood that nucleotides may differ in the indicated number but may still have similar neighboring nucleotides. Said nucleotides which may be exchanged, deleted or comprise additional nucleotides are also comprised by the term "corresponding position".

The position with respect to nucleotide sequences mentioned herein refers to the sequence shown in SEQ ID NO: 1 (Figures 4A to C). This sequence represents the genomic nucleic acid sequence of the ATGL gene encoding the adipose triglyceride lipase. It is possible for the skilled person to identify the position in the genomic sequence corresponding to a position in SEQ ID NO: 1 by aligning the sequences. Moreover, the exact locations of the exons and introns are indicated in SEQ ID NO: 1 hereinbelow. Additionally, the person skilled in the art is able to identify exons and introns of the ATGL gene by comparing SEQ ID NO: 1 with SEQ ID NO: 2 which shows the cDNA sequence of the ATGL gene.

In order to determine whether an amino acid residue or nucleotide residue in a given ATGL sequence corresponds to a certain position in the amino acid sequence or nucleotide sequence of SEQ ID NO: 1, 2 or 3 (Figures 4A to C, Figures 5 and 6, respectively), the skilled person can use means and methods well-known in the art, e.g., alignments, either manually or by using computer programs such as those mentioned further down below in connection with the definition of the term "hybridization" and degrees of homology.

For example, BLAST2.0, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments. BLAST produces alignments of both nucleotide and amino acid sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cutoff score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\%\text{sequence identity} \times \%\text{ maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules. Another example for a program capable of generating sequence alignments is the CLUSTALW computer program (Thompson, Nucl. Acids Res. 2 (1994), 4673-4680) or FASTDB (Brutlag Comp. App. Biosci. 6 (1990), 237-245), as known in the art.

As described herein, the present disclosure describes several SNPs in the ATGL gene which means that at a certain position of the wild-type ATGL nucleotide sequence a nucleotide is replaced by a different nucleotide as described herein. The term "ATGL gene" includes the 5'UTR, exons, introns and the 3'UTR of ATGL whose genomic wild-type nucleotide sequences is shown in SEQ ID NO: 1 (Figures 4A to C). The term "is replaced by a different nucleotide" when used in the context of the present invention means that the nucleotide residing at a position of the wild-type ATGL nucleic acid sequences mentioned herein is replaced by a nucleotide which is not the same nucleotide as that residing on the said position of the wild-type ATGL nucleic acid sequences. For example, if a cytidine resides at position X of the wild-type ATGL nucleic acid sequence, then it is not replaced by cytidine, but, for example, replaced by guanidine, adenine or thymidine.

In the following, the SNPs found in the ATGL gene are described. A first group of SNPs resides in the 5'UTR of the ATGL gene.

Preferably, at position -2210 (also designated as "SNP 1" shown in Table 2 below) in the 5'UTR of the genomic sequence of the ATGL gene depicted in SEQ ID NO: 1 (Figures 4A to C) a thymine (T) is replaced by a different nucleotide, preferably a pyrimidine base. More preferably, at said position said thymidine is replaced by a purine base, such as adenosine (A) or guanosine (G). Particularly preferred, said thymine is replaced by an adenosine (A).

Preferably, at position -2014 (also designated as "SNP 2" shown in Table 2 below) in the 5'UTR of the genomic sequence of the ATGL gene depicted in SEQ ID NO: 1 (Figures 4A to C) a guanosine (G) is replaced by a different nucleotide, preferably a pyrimidine base, such as cytosine (C) or thymine (T). More preferably, at said position said guanosine is replaced by another purine base, such as adenosine (A). Particularly preferred, said guanosine is replaced by adenosine (A).

A second type of mutation found in the ATGL gene are mutations in exons which may or may not lead to amino acid substitutions in the corresponding amino acid sequence. For example, mutations at the third position of a triplet codon, i.e. at the wobble base, lead to so-called silent mutations. Silent mutations do normally not lead to a change of the amino acid due to the degeneracy of the genetic code, i.e. 64 triplets encode at all 20 naturally occurring amino acids. However, said silent mutations lead to a change in the codon encoding its respective amino acids insofar that the newly generated codon may not fit so well into the codon usage of an organism. Namely, the newly generated codon is not translated by the ribosome with the same efficiency as the "old" codon. This may lead to insufficient amounts of the corresponding polypeptide causing an distinct phenotype.

Mutations in exons of the ATGL gene are listed under item 1(b), Table A or item 2(a), Table C, supra. At the respective position indicated in the column "Exon" which is replaced by another nucleotide residue is shown. Accordingly, the term "a nucleotide as indicated in column "Exon" of the Table A corresponding to the position as indicated in column "Replaced nucleotide" of Table A corresponding to the position as indicated in column "Position in wild-type" of Table A is replaced by a different nucleotide means that a nucleotide residue in a ATGL encoding sequence would be located at position Y in SEQ ID NO: 1 (Figures 4A to C) when the ATGL coding sequence including all exons of the ATGL gene is compared and aligned with the exon sequences of the genomic ATGL sequence shown in SEQ ID NO: 1 (Figures 4A to C) or the ATGL cDNA sequence shown in SEQ ID NO: 2 (Figure 5).

If the nucleotide at the respective position is a pyrimidine base such as cytosine or thymidine it is preferred that due to a transition it is replaced by another pyrimidine base.

For example, a cytosine is replaced by a thymidine or a thymidine is replaced by a cytosine. If the nucleotide at the respective position is a purine base it is preferred that due to a transition it is replaced by another purine base. For example, adenosine is replaced by guanosine or guanosine is replaced by adenosine.

It is also preferred that due to a transversion a purine base is replaced by a pyrimidine base or vice versa. For example, an adenine is replaced by a thymine or vice versa or a guanine is replaced by a cytidine or vice versa.

Preferably, in exon 5 at position 2847 (also designated as "SNP4" shown in Table 2 below) of the ATGL sequence shown in SEQ ID NO: 1 (Figures 4A to C) a cytidine is replaced by another nucleotide. For example, it could be replaced by a purine base, such as adenosine or guanosine. Preferably, said cytidine at position 2847 is replaced by a pyrimidine base, preferably, thymidine. In case, a nucleotide in one of the exon mutations described herein is changed to another nucleotide, this may create a codon which encodes a different amino acid. "Different" means that the amino acid present in the wild-type ATGL amino acid sequence is changed to an amino acid which is different from the wild-type amino acid at the respective position.

Preferably, in exon 5 at position 2870 (also designated as "SNP5" shown in Table 2 below) of the ATGL sequence shown in SEQ ID NO: 1 (Figures 4A to C) a cytidine is replaced by another nucleotide. For example, it could be a purine base, such as adenosine or guanosine.

Preferably, said cytidine at position 2847 or 2870 is replaced by a pyrimidine base, preferably, thymidine.

Preferably, in exon 6 at position 3871 (also designated as "SNP7" shown in Table 2 below) of the ATGL sequence shown in SEQ ID NO: 1 (Figures 4A to C) a cytidine is replaced by another nucleotide. For example, it could be replaced by a pyrimidine base, such as thymidine. Preferably, said cytidine at position 3871 is replaced by a purine base, such as adenosine, preferably guanosine.

Preferably, in exon 7 at position 4091 (also designated as "SNP8" shown in Table 2 below) of the ATGL sequence shown in SEQ ID NO: 1 (Figures 4A to C) a cytidine is replaced by another nucleotide. For example, it could be replaced by a pyrimidine base, such as thymidine. Preferably, said cytidine at position 4091 is replaced by a purine base, such as adenosine, preferably guanosine.

Preferably, at position 4710 (also designated as "SNP9" shown in Table 2 below) of the ATGL sequence shown in SEQ ID NO: 1 (Figures 4A to C) a guanosine is replaced by another nucleotide. For example, it could be replaced by a purine base, such as adenosine. Preferably, said guanosine is replaced by a pyrimidine base, such as cytidine, preferably, thymidine.

Preferably, at position 5071 (also designated as "SNP10" shown in Table 2 below) of the ATGL sequence shown in SEQ ID NO: 1 (Figures 4A to C) a cytidine is replaced by another nucleotide. For example, it could be replaced by a purine base, such as adenosine or guanosine. Particularly preferred, said cytidine at position 2847 or 5071 is replaced by a pyrimidine base, preferably, thymidine.

Another group of mutation (mentioned in item 1 (e), Table B supra) resides in introns of the ATGL gene, in particular in intron 2 or 5 of the genomic sequence of the ATGL gene shown in SEQ ID NO: 1 (Figures 4A to C). Said mutations in said introns are point mutations.

At the respective position indicated in the column "Position in wild-type" in Table B the position of the nucleotide residue in the respective intron which is replaced by another nucleotide residue is shown. Accordingly, the term "a nucleotide as indicated in column "Intron" of the Table B corresponding to the position as indicated in column "Replaced nucleotide" of Table B corresponding to the position as indicated in column "Position in wild-type" of Table B is replaced by another nucleotide means that a nucleotide residue in the ATGL sequence would be located at position Y in SEQ ID NO: 1 (Figures 4A to C) when the ATGL sequence is compared and aligned with the sequence of SEQ ID NO: 1.
If the nucleotide at the respective position is a pyrimidine base it is preferred that due to a transition it is replaced by another pyrimidine base. For example, thymine is replaced by a cytidine or a cytidine is replaced by a thymine.

It is also preferred that due to a transversion a pyrimidine base is replaced by a purine base. For example, a cytidine or thymidin is replaced by an adenine or guanidine or vice versa . Preferably, at position 550 (also designated as "SNP3" shown in Table 2 below) of the ATGL sequence shown in SEQ ID NO: 1 (Figures 4A to C), a guanidine is replaced by another nucleotide. Preferably, at position 550 a guanidine is replaced by a pyrimidine base, such as thymidine or cytidine. Preferably, at position 550 a guanidine is replaced by another purine base, preferably, adenine. Preferably, at position 2904 (also designated as "SNP6" shown in Table 2 below) of the ATGL sequence shown in SEQ ID NO: 1, an adenine is replaced by another nucleotide. For example, said adenine at position 2904 is replaced by a pyrimidine, such as thymidine or cytidine. Preferably, said adenine at position 2904 is replaced by another purine, preferably, guanine.

Another group of mutations in the ATGL gene described hereinabove in item (c) is a deletion of preferably 4 base pairs corresponding to positions 5274 to 5277 (also designated as "SNP11" shown in Table 2 below) of the wild-type genomic ATGL nucleotide sequence acid as shown in SEQ ID NO: 1 (Figures 4A to C). However, also deletion mutants are comprised in which either more or less nucleotides within the ATGL nucleotide sequence set forth in SEQ ID NO: 1 may be deleted due to, for example, atypical splicing or deletion of nucleotides of the nucleic acid molecule encoding ATGL or wrong posttranscriptional processes. Thus, it is envisaged that at least one nucleotide is deleted upstream of a position corresponding to position 5274 of the genomic nucleotide sequence of the wild-type ATGL as shown in SEQ ID NO: 1 It is also envisaged that at least one nucleotide downstream of a position corresponding to position 5277 is deleted. The number of deleted nucleotides is n, wherein n is an integer in the range of 1 to ∞, preferably 1 to 500, 400, 300, 200, 100, 50, 40, 30, 25, 20, 15, 10, 9, 8, 7, 6, 5, 4, 3 or 2 .

A further group of mutations in the ATGL gene described hereinabove in item (d) is an addition of at least one nucleotide starting at position corresponding to position 5274 (also designated as "SNP11" shown in Table 2 below) of the genomic nucleotide sequence of the wild-type ATGL as shown in SEQ ID NO: 1 (Figures 4A to C). Thus, it is envisaged that at least one nucleotide is added upstream of a position corresponding to position 5274 of the genomic nucleotide sequence of the wild-type ATGL as shown in SEQ ID NO: 1 is added. The number of added nucleotides is n, wherein n is an integer in the range of 1 to ∞, preferably 1 to 25, 20, 15, 10, 9, 8, 7, 6, 5, 4, 3, 2 or 1. Preferably, the number of added nucleotides is 4.

A last group of mutations that has been identified relates to mutations which reside in the 3'UTR of the wild-type ATGL gene depicted in SEQ ID NO: 1 (Figures 4A to C). The mutation was found at positions 5392 (also designated as "SNP12" shown in Table 2 below) of the wild-type ATGL gene shown in SEQ ID NO: 1. Preferably, at said position 5392 a guanidine is replaced by another nucleotide, e.g. a pyrimidine base, such as cytidine or thymidine. More preferably, at said position 5392 a guanidine is replace by another purine base, preferably adenine.

The following **Table 1** shows haplotypes, haplotype frequency, difference in FFA levels from haplotype association analysis via linear regression model adjusted for age and sex.

| Haplotypes | | Frequency± SE[*] | FFA difference for 1 (2) copies of the haplotype | P-value |
|---|---|---|---|---|
| SNP- Number | 1 1 1 1 2 3 5 6 7 8 9 0 1 2 | | | |
| H1 | 2-1-1-1-2-1-1-1-1-2 | 0.134 ± 0.005 | -0.50 (-0.24) | 0.03[#] |
| H2 | 2-1-1-1-2-2-1-1-1-2 | 0.010 ± 0.001 | -1.07 | 0.02 |
| H3 | 1-2-1-1-1-1-1-1-1-1 | 0.034 ± 0.003 | -0.36 | 0.22 |
| H4 | 1-1-1-1-1-1-1-1-1-2 | 0.024 ± 0.002 | -0.66 | 0.03 |
| H5 | 1-1-1-1-1-1-1-1-1-1 | 0.514 ± 0.007 | reference | - |
| H6 | 1-1-1-2-2-1-1-2-1-2 | 0.005 ± 0.001 | +1.18 | 0.20 |
| H7 | 1-1-2-1-2-1-1-1-2-2 | 0.007 ± 0.001 | -1.06 | 0.33 |
| H8 | 1-1-2-1-2-1-1-1-2-2-2 | 0.024 ± 0.002 | -0.75 | 0.04 |
| H9 | 1-1-2-1-2-1-2-1-2-2-2 | 0.221 ± 0.006 | -0.43 (-0.99) | 0.0009[##] |

* expected haplotype frequency and standard error;
# not assuming a trend per copy., ## assuming a trend per copy

The following **Table 2** shows the investigated polymorphism at the ATGL locus.

| Number | Position[a] | Variation | Location | % Minor allele | % Heterozygosity |
|---|---|---|---|---|---|
| 1 | -2210 | T/A | Promoter | 14.6 | 25.2 |
| 2 | -2014 | G/A | Promoter | 3.8 | 7.3 |
| 3 | +550 | G/A | Intron2 | 26.3 | 37.9 |
| 4 | +2847 | C/T | Exon5 L219F | 0.02 (n=1) | - |
| 5 | +2870 | C/T | Exon5 L226L | 0.6 | 1.1 |
| 6 | +2904 | A/G | Intron5 | 41.5 | 47.9 |
| 7 | +3871 | C/G | Exon6 N252K | 1.0 | 2.0 |
| 8 | +4091 | C/G | Exon7 P291 P | 23.2 | 34.6 |
| 9 | +4710 | G/T | Exon9 L389L | 0.9 | 1.5 |
| 10 | +5071 | C/T | Exon10 P481L | 26.0 | 37.5 |
| 11[b] | +5274 | TGCA/* | 3'UTR | 26.3 | 37.5 |
| 12 | +5392 | G/A | 3'UTR | 44.2 | 49.0 |

a relating to the first position of the translation starting point ATG
b relating to a SNP in which nucleotides are deleted or added as described hereinabove

As is evident from the above; not all identified mutations are located in exons or lead to a change in the amino acid sequence. Some of the mutations are located in the 5'UTR, the 3'UTR or in introns.

It is known that polymorphisms in promoter and enhancer regions can affect gene function by modulating transcription, particularly if they are situated at recognition sites for DNA binding proteins (Fishman et al., J. Clin. Invest. 102 (1998), 1369-1376). The term "polymorphism" which is used in the present invention means single nucleotide substitution, nucleotide insertion and nucleotide deletion which in the case of insertion and deletion includes insertion or deletion of one or more nucleotides at a position of a gene and corresponding alterations in expressed proteins. Polymorphisms in the 5' untranslated region (5'UTR) of genes can affect the efficiency with which proteins are translated. A representative example of this is in the c-myc gene where a C-G SNP that creates an internal ribosome entry site is associated with increased efficiency of c-myc translation and myeloma (Chappell et al., Oncogene 19 (2000), 4437-4440). Polymorphisms in the 3'UTR can affect gene function by altering the secondary structure of RNA and efficiency of translation or by affecting motifs in the RNA that bind proteins which regulate RNA degradation. Polymorphisms within introns can affect gene function by affecting RNA splicing resulting in aberrant polypeptides. Another way in which intronic polymorphisms can affect gene function is when they affect regulatory motifs within introns. Examples are the Sp1 binding site polymorphism within intron 1 of the COLIAL gene (Mann et al., J. Clin. Invest 107 (2001), 899-907) and a repeat polymorphisms within the IL-1Ra gene (Keen et al., Bone 23 (1998), 367-371). Further examples between intronic SNPs and gene function are described in Caceres and Kornblihtt, Trends Genet. 4 (2002), 186-93.

The present invention also relates to nucleic acid molecules which hybridize to one of the above described nucleic acid molecules and which shows a mutation as described hereinabove.

The term "hybridizes" as used in accordance with the present invention may relate to hybridizations under stringent or non-stringent conditions. If not further specified, the conditions are preferably non-stringent. Said hybridization conditions may be established according to conventional protocols described, for example, in Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as 0.1xSSC, 0.1% SDS at 65°C. Non-stringent hybridization conditions for the detection of homologous or not exactly complementary sequences may be set at 6xSSC, 1% SDS at 65°C. As is well known, the length of the probe and the composition of the nucleic acid to be determined constitute further parameters of the hybridization conditions. Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Hybridizing nucleic acid molecules also comprise fragments of the above described molecules. Such fragments may represent nucleic acid sequences which code for a non-functional ATGL or a non-functional fragment thereof, and which have a length of at least 12 nucleotides, preferably at least 15, more preferably at least 18, more preferably of at least 21 nucleotides, more preferably at least 30 nucleotides, even more preferably at least 40 nucleotides and most preferably at least 60 nucleotides. Furthermore, nucleic acid molecules which hybridize with any of the aforementioned nucleic acid molecules also include complementary fragments, derivatives and allelic variants of these molecules. Additionally, a hybridization complex refers to a complex between two nucleic acid sequences by virtue of the formation of hydrogen bonds between complementary G and C bases and between complementary A and T bases; these hydrogen bonds may be further stabilized by base stacking interactions. The two complementary nucleic acid sequences hydrogen bond in an antiparallel configuration. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed). The terms complementary or complementarity refer to the natural binding of polynucleotides under permissive salt and temperature conditions by base-pairing. For example, the sequence "A-G-T" binds to the complementary sequence "T-C-A". Complementarity between two single-stranded molecules may be "partial", in which only some of the nucleic acids bind, or it may be complete when total complementarity exists between single-stranded molecules. The degree of complementartity between nucleic acid strands has significant effects on the efficiency and strength of hybridization between nucleic acid strands. This is of particular importance in amplification reactions, which depend upon binding between nucleic acids strands.

The term "hybridizing sequences" preferably refers to sequences which display a sequence identity of at least 40%, preferably at least 50%, more preferably at least 60%, even more preferably at least 70%, particularly preferred at least 80%, more particularly preferred at least 90%, even more particularly preferred at least 95% and most preferably at least 97% identity with a nucleic acid sequence as described above encoding an ATGL protein having a described mutation. Moreover, the term "hybridizing sequences" preferably refers to sequences encoding an ATGL protein having a sequence identity of at least 40%, preferably at least 50%, more preferably at least 60%, even more preferably at least 70%, particularly preferred at least 80%, more particularly preferred at least 90%, even more particularly preferred at least 95% and most preferably at least 97% identity with an amino acid sequence of an ATGL mutant as described herein above.

In accordance with the present invention, the term "identical" or "percent identity" in the context of two or more nucleic acid or amino acid sequences, refers to two or more sequences or subsequences that are the same, or that have a specified percentage of amino acid residues or nucleotides that are the same (e.g., 60% or 65% identity, preferably, 70-95% identity, more preferably at least 95% identity), when compared and aligned for maximum correspondence over a window of comparison, or over a designated region as measured using a sequence comparison algorithm as known in the art, or by manual alignment and visual inspection. Sequences having, for example, 60% to 95% or greater sequence identity are considered to be substantially identical. Such a definition also applies to the complement of a test sequence. Preferably the described identity exists over a region that is at least about 15 to 25 amino acids or nucleotides in length, more preferably, over a region that is about 50 to 100 amino acids or nucleotides in length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul Nucl. Acids Res. 25 (1977), 3389-3402). The BLASTN program for nucleic acid sequences uses as defaults a word length (W) of 11, an expectation (E) of 10, M=5, N=4, and a comparison of both strands. For amino acid sequences, the BLASTP program uses as defaults a wordlength (W) of 3, and an expectation (E) of 10. The BLOSUM62 scoring matrix (Henikoff Proc. Natl. Acad. Sci., USA, 89, (1989), 10915) uses alignments (B) of 50, expectation (E) of 10, M=5, N=4, and a comparison of both strands.

Moreover, the present invention also relates to nucleic acid molecules the sequence of which is degenerate in comparison with the sequence of an above-described hybridizing molecule. When used in accordance with the present invention the term "being degenerate as a result of the genetic code" means that due to the redundancy of the genetic code different nucleotide sequences code for the same amino acid.

The present invention also relates to nucleic acid molecules encoding ATGL and which comprise one or more of the above-described mutations.

In a further aspect, the present invention relates to a diagnostic composition comprising one or more of the nucleic acid sequences as defined herein above.

The diagnostic composition optionally comprises suitable means for detection. The nucleic acid molecule(s), or polypeptide(s) described above are, for example, suitable for use in immunoassays in which they can be utilized in liquid phase or bound to a solid phase carrier. Examples of well-known carriers include glass, polystyrene, polyvinyl ion, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Solid phase carriers are known to those in the art and may comprise polystyrene beads, latex beads, magnetic beads, colloid metal particles, glass and/or silicon chips and surfaces, nitrocellulose strips, membranes, sheets, duracytes and the walls of wells of a reaction tray, plastic tubes or other test tubes. Suitable methods of immobilizing nucleic acid molecule(s), vector(s), host(s), antibody(ies), aptamer(s), polypeptide(s), etc. on solid phases include but are not limited to ionic, hydrophobic, covalent interactions or (chemical) crosslinking and the like. Examples of immunoassays which can utilize said compounds of the invention are competitive and non-competitive immunoassays in either a direct or indirect format. Commonly used detection assays can comprise radioisotopic or non-radioisotopic methods. Examples of such immunoassays are the radioimmunoassay (RIA), the sandwich (immunometric assay) and the Northern or Southern blot assay. Furthermore, these detection methods comprise, inter alia, IRMA (Immune Radioimmunometric Assay), EIA (Enzyme Immuno Assay), ELISA (Enzyme Linked Immuno Assay), FIA (Fluorescent Immuno Assay), and CLIA (Chemioluminescent Immune Assay). Furthermore, the diagnostic compounds of the present invention may be are employed in techniques like FRET (Fluorescence Resonance Energy Transfer) assays.

Appropriate labels and methods for labeling are known to those of ordinary skill in the art. Examples of the types of labels which can be used in the present invention include inter alia, fluorochromes (like fluorescein, rhodamine, Texas Red, etc.), enzymes (like horse radish peroxidase, β-galactosidase, alkaline phosphatase), radioactive isotopes (like 32P, 33P, 35S or 125I), biotin, digoxygenin, colloidal metals, chemi- or bioluminescent compounds (like dioxetanes, luminol or acridiniums).

A variety of techniques are available for labeling biomolecules, are well known to the person skilled in the art and are considered to be within the scope of the present invention and comprise, inter alia, covalent coupling of enzymes or biotinyl groups, phosphorylations, biotinylations, random priming, nick-translations, tailing (using terminal transferases). Such techniques are, e.g., described in Tijssen, "Practice and theory of enzyme immunoassays", Burden and von Knippenburg (Eds), Volume 15 (1985); "Basic methods in molecular biology", Davis LG, Dibmer MD, Battey Elsevier (1990); Mayer, (Eds) "Immunochemical methods in cell and molecular biology" Academic Press, London (1987); or in the series "Methods in Enzymology", Academic Press, Inc.

Detection methods comprise, but are not limited to, autoradiography, fluorescence microscopy, direct and indirect enzymatic reactions, etc.

Said diagnostic composition may be used for methods for detecting the presence and/or abundance of a nucleic acid molecule of the invention in a biological and/or medical sample and/or for detecting expression of such a nucleic acid molecule (e.g. by determining the mRNA or the expressed polypeptide). Furthermore, said diagnostic composition may also be used in methods of the present invention, inter alia, for the detection of specific antagonists or agonists for ATGL (see herein below). Additionally, the present invention relates to a kit comprising the diagnostic composition as described herein or the nucleic acid molecule, the polypeptide the primer or pair of primers of the invention (the mentioned primers are described in the appended examples; see supplementary Table A and B) or the molecule as identified or characterized in a method herein below of the present invention.

Advantageously, the kit of the present invention further comprises, optionally (a) reaction buffer(s), storage solutions and/or remaining reagents or materials required for the conduct of scientific or diagnostic assays or the like. Furthermore, parts of the kit of the invention can be packaged individually in vials or bottles or in combination in containers or multicontainer units.

The kit of the present invention may be advantageously used as diagnostic kits, as research tools or therapeutic tools.

Additionally, the kit of the invention may contain means for detection suitable for scientific, medical and/or diagnostic purposes. The manufacture of the kits follows preferably standard procedures which are known to the person skilled in the art.

Accordingly, the present invention relates to the following basic teaching: Adipose triglyceride lipase (ATGL) was recently described to predominantly perform the initial step in triglyceride hydrolysis (Science 306:1383, 2004) and therefore seems to play a pivotal role in the lipolytic catabolism of stored fat in adipose tissue. This is the first study which investigates genetic variations within the ATGL gene in humans.

We investigated in a large epidemiological study involving 2434 Caucasians from Utah, USA, twelve polymorphisms in the ATGL gene identified via sequencing and data base search. These polymorphisms and their statistically reconstructed haplotypes were analysed for association with plasma free fatty acids (FFA) and other phenotypes related to lipoprotein metabolism, obesity, and type 2 diabetes. FFA concentrations were significantly associated with several SNPs and haplotypes of the ATGL gene (decreased FFA levels: SNPs 3,6,8,10,11,12, p-values from 0.016 to 0.00007), consistent with additive inheritance. Three rare SNPs (SNPs 5,7,9) showed a tendency towards significance. Furthermore, SNP5 (and similarly SNP9) showed associations with glucose levels (p=0.00017, +19 mg/mL), risk of type 2 diabetes (OR=2.6, p=0.025) and nonsignificant but consistent differences in BMI and HOMA. Triglyceride levels showed a nonsignificant tendency for an association with ATGL in accordance with the FFA associations. The associations of triglycerides and glucose became stronger when restricting the analysis to subjects without diabetic medication. Haplotype analysis supported and extended the shown SNP association analyses.

In summary, genetic variation within the ATGL gene shows strong associations with FFA, and less pronounced but still detectable associations with parameters of the metabolic syndrome and type 2 diabetes.

The figures show:

| | |
|---|---|
| **Figure 1:** | Genetic structure of the *ATGL* gene including the genotyped polymorphism. The top is a schematic of the *ATGL* gene with the SNP locations. The shade of the diamond represents the pair-wise $r^2$ between the two SNPs defined by the top left and the top right sides of the diamond. Shading represents magnitude and significance of the pair-wise $r^2$, with black reflecting high $r^2$ (>0.8) and white reflecting low $r^2$ (<0.20). , |
| **Figure 2:** | (a) Primary analysis: association of the 11 genotyped polymorphisms of the *ATGL* gene indicating mean changes in plasma concentrations of free fatty acids (FFAs) for subjects with one or two copies of the minor allele compared to subjects with the wildtype (SNP 4 discarded as observed only in one individual). Results are derived from a linear regression model adjusting for age and sex and a log-transformed outcome. The stars mark statistical significance to 0.05 level or to a level corrected for 9.1 effective loci (*, p<0.05; and **, p<0.006). (b) and (c) Secondary analyses: same as (a) for triglyceride and glucose plasma concentrations, respectively. (d) Summary of p-values testing the association with and without assuming an additive inheritance model (i.e. with p-values assuming a trend per copy of the minor allele or overall p-values). |
| **Figure 3:** | Minimal spanning net for haplotypes: starting from the most common haplotype, the cascade indicates distance of each haplotype in terms of number of mutations away from the most common haplotype. Also stated are frequencies, the change in FFAs for subjects carrying one or two haplotypes compared to the reference together with a p-value testing for significance (for log FFAs) assuming a trend per haplotype copy where appropriate. |
| **Figures 4A to 4C:** | ATGL (adipose triglyceride lipase) The sequence depicted in Figure 4 represents **SEQ ID NO: 1** The genetic information is derived from Ensembl database PNPLA2 (patatin-like phospholipase domain containing 2, ENSG00000177666); This gene can be found on Chromosome 11 at location: <u>808,902-815,216</u> ; the start of this gene is located in <u>Contig AP006621.1.1.129989.</u>' |
| **Figure 5:** | cDNA-sequence of ATGL (exon transitions are coloured) - The sequence depicted in Figure 5 represents **SEQ ID NO: 2** |
| **Figure 6:** | The sequence depicted in Figure 6 represents **SEQ ID NO: 3 -** protein sequence of ATGL |
| **Figures 7 A and B:** | single SNPs with sequence, exchange and possible influence on the encoded amino acid - the sequences depicted in Figure 7 represent **SEQ ID Nos: 4 to 15,** respectively |

## References

1. Boden,G. & Shulman,G.I. Eur. J. Clin. Invest 32 Suppl 3, 14-23 (2002).
2. Zechner,R., Strauss,J.G., Haemmerle,G., Lass,A., & Zimmermann,R. Curr. Opin. Lipidol. 16, 333-340 (2005).
3. Zimmermann,R. et al. Science 306, 1383-1386 (2004).
4. Jenkins,C.M. et al. J. Biol. Chem. 279, 48968-48975 (2004).
5. Villena,J.A., Roy,S., Sarkadi-Nagy,E., Kim,K.H., & Sul,H.S. J. Biol. Chem. 279, 47066-47075 (2004).
6. Nyholt,D.R. Am. J. Hum. Genet. 74, 765-769 (2004).
7. Miranda,P.J., DeFronzo,R.A., Califf,R.M., & Guyton,J.R. Am. Heart J. 149, 33-45 (2005).
8. Lam,T.K. et al. Am. J. Physiol Endocrinol. Metab 284, E863-E873 (2003).

## Examples

The following examples illustrate the invention. These examples should not be construed as to limit the scope of this invention. The examples are included for purposes of illustration and the present invention is limited only by the claims.

## Example 1: Study population

The entire analyzed study population (n=2434) consisted of three groups of subjects (Supplementary Table D). First, 1101 subjects with morbid obesity (BMI >35 kg/m$^2$) who where either seeking gastric bypass surgery (GBS) or subjects who were randomly chosen from a population of severely obese participants not seeking GBS. Second, 469 premature, familial coronary heart disease (CHD) cases who had survived a myocardial infarction, percutaneous transluminal angioplasty, or coronary artery bypass grafting before age 55 for men or age 65 for women. To minimize artefactual effects of the acute coronary syndrome on lipid or FFA levels, patients were seen at least 6 months after their acute event. Third, controls consisted of 864 individuals from the same geographical region and found to be representative of the Utah population [1]. CHD cases and controls are described in detail elsewhere as part of a recent study [2].

## Example 2: Laboratory phenotyping

Blood samples were collected after an overnight fasting period. Subjects were only allowed to drink water for 12 hours prior to their clinic visit. Plasma free fatty acids (FFAs) were measured using an enzymatic colorimetric assay from Wako (Richmond, VA). Triglyceride concentrations were measured with a Roche FARA II (Roche Diagnostics, Indianapolis, Indiana) automated analyzer.

## Example 3: Sequencing

Polymorphism detection in the human *ATGL* gene was done by sequence-analysis of defined PCR products in the promoter region and in all exons and introns (except 368 bp of intron1/2 and 440 bp of intron 2/3) in 48 normal weight (BM=20-25 kg/m$^2$) and 48 obese individuals (BMI=35-90 kg/m$^2$). Thirteen overlapping PCR products covered the promoter region, and the whole gene from -3290 bp up to 5642 bp according to NM_020376 of the Ensembl database. All PCR and sequencing primers were designed using the Vector NTI suite 9.0 [www.informaxinc.com] (Supplementary Tables A and B). Cycle sequencing of the purified PCR products was performed on the ABI prism 3730 and ABI prism 3130 Genetic Analyser following a standardized protocol (Applied Biosystems, Foster City, CA, USA).

## Example 4: SNP selection and Genotyping of the SNPs

From the SNPs detected by sequencing the selection for genotyping in the entire group was mostly guided by potential functional considerations. Thereby, we selected 11 SNPs for the following reasons ordered by decreasing priority: resulting amino acid exchange, location at the intron-exon boundary, location in the untranslated 3'UTR region, minor allele frequency above 5%, or being a tagging SNP. In addition, we selected one 4 basepair insertion/deletion polymorphism in position +5274. Selection of tagging SNPs was performed using the tagSNP program by Stram and colleagues [3].
Supplementary Table C describes the 12 polymorphisms known from public databases and from re-sequencing selected for genotyping. All SNPs were genotyped by MALDI TOF MS system, (Sequenom, Mass EXTEND, San Diego, USA) as described earlier [4]. The insertion/deletion polymorphism (+5274) was genotyped by TaqMan using an ABI Prism 7900 HT (Applied Biosystems, Foster City, CA, USA) and TaqMan MGB "assay by design" reagents. The genotyping success rate of all 12 polymorphisms was above 98%.

## Example 5: Analyzed sample

The original group included 2459 individuals. Twenty-five subjects were excluded from the analysis due to missing age (n=12) or if at least 6 of the 12 SNPs were unsuccessfully genotyped (n=13) leaving 2434 subjects in the analysis. Association analysis of genetic variations in the *ATGL* gene with T2DM was done in these study participants. Fasting triglyceride and glucose levels were available for 2426 and 2429 subjects, respectively. Plasma for measurement of FFA levels sampled and stored at -80°C in appropriate conditions was available in 1922 individuals. The sample for the analysis of these three plasma levels was further restricted to subjects not taking any diabetes medication known to modulate FFA, triglycerides and glucose concentrations leaving 2190, 2193 and 1701 subjects in the analysis. Statistical haplotype reconstruction was performed in 2228 subjects in whom genotypes of all 11 polymorphisms were available. The haplotype association analysis with FFA, triglyceride, and glucose concentrations was performed in 1563, 2008, and 2010 subjects, respectively.

## Example 6: Statistical association analysis

Genotype and allele frequencies were derived and a test of Hardy-Weinberg equilibrium was performed. The relationship between the SNPs was summarized by the correlation coefficient and Lewontin's D'. The haplotypes were estimated based on the 11 SNPs available for all 2228 subjects via the expectation-maximization algorithm by the SAS® procedure PROC HAPLOTYPE (version 9.0, release 2004) and by using PHASE [5]. Both algorithms yielded the same results and the portion of unambiguously defined haplotypes was very high (99%). Rare haplotypes with frequency <0.5% were summarized into one group. SNP4 was observed only in one subject and was not considered in any of the statistical analyses.

The primary association analysis was a general linear regression model adjusted for age and gender with logarithm of free fatty acids (FFAs) as the outcome. The difference of the means of the logarithm of the FFA levels for subjects with 0, 1 or 2 copies of the minor allele of a SNP was tested for each SNP separately with and without assuming a trend per copy of the minor allele. Haplotype association analysis was carried out with the same statistical model, testing the change of log FFA levels for each copy of a haplotype against the reference (i.e. the group of subjects with two copies of the most common haplotype). A possible effect of the sample origin (GB, CHD, controls) was accounted for by adjusting the analysis for the subgroup or by performing a sensitivity analysis excluding one group after another.

In a secondary analysis, the same statistical procedure as described above for FFA was applied for triglyceride and glucose levels which are expected to be related to FFA metabolism. Association of the SNPs with occurrence of type 2 diabetes (T2DM) was tested via logistic regression.

**Supplementary Table A:** PCR reactions used for SNP screening

| Primer ID | 1st PCR Primer | 2nd PCR Primer | Amplification Product [bp] | Q-Solution | Annealing Temperature | Annealing Time | Extension Time |
|---|---|---|---|---|---|---|---|
| PCR1 | TGACGAGCCCCTGGACTTCCAT (SEQ ID NO: 16) | GTTGAGGTGAGTGGATCACGAGG (SEO ID NO: 17) | -3315 - -2299 | yes | 67° | 45s | 1:30m |
| PCR 2 | GACCTCGTGATCCACTCACC(SEQ ID NO: 18 | TAGTGCACGCATGTAGTCCTAG (SEQ ID NO: 19) | -2223--1716 | yes | 69° | 45s | 1:30m |
| PCR 3 | CCCCACACGTGGTTACCTAC (SEQ ID NO: 20) | CCAAGCTGAGTTAGGGAAGC (SEQ ID NO: 21) | -1917--1110 | no | 61° | 30s | 1:00m |
| PCR 4 | ATGTAAATGCCTACCGGCC (SEQ ID NO: 22) | GACCCATAAACATCCAAGGC(SEQ ID NO: 23) | -1267--617 | yes | 57° | 45s | 1:30m |
| PCR 5 | CTGGGGAGACAGAATCTCATTO (SEQ ID NO: 24) | CGAGATGTTCCACGTCTTCTC(SEQ ID NO: 25) | -1017 - 33 | yes | 57° | 45s | 1:30m |
| PCR 6 | TAGCTTCTTCGCCTCCGCCA (SEQ ID NO: 26) | GCTCACCCAGGCAGACCCCG(SEQ ID NO: 27) | -148 - 193 | yes | 69.5° | 45 | 1:00m |
| PCR 7 | CATGGGGGTAGCCTCTTGAG (SEQ ID NO: 28) | CAAACACTCCTCTTGGGGC(SEQ ID NO: 29) | 605 - 1425 | no | 64° | 30s | 1:00m |
| PCR 8 | CACTCCTGACCAGGGTGATG (SEQ ID NO: 30) | ATGAGCAGAGGCCTTGAGG(SEQ ID NO: 31) | 1300 - 2211 | no | 63° | 30s | 1:00m |
| PCR 9 | AGTGCGAACCTCAAGGCCTC (SEQ ID NO: 32) | AGAGAGAGACTGGACAGGGAGC (SEO ID NO: 33) | 2185 - 3101 | yes | 68° | 45s | 1:30m |
| PCR 10 | TTCCCAGCCACTCCTCACTG (SEO ID NO: 34) | TCTCAGTTCATCAGCGCGGC (SEO ID NO: 35) | 2918 - 3955 | no | 66° | ID 30s | 1:00m |
| PCR 11 | GGCCGCGCTGATGAACTGAGAATC (SEO ID NO: 36) | CAGCATGTTGGAGAGGGTGGTCAG (SEQ ID NO: 37) | 3935 - 4341 | yes | 68° | 45s | 1:30m |
| PCR 12 | GAAAGAGAGAGAGGAGAGGACG (SEO ID NO: 38) | CACGTTGGTGCAGAAGAGG(SEQ ID NO: 39) | 4184 - 4988 | yes | 62° | 45s | 1:30m |
| PCR 13 | CGAGGACATCCGGTGGATGA (SEQ ID NO: 40) | AAATGCCAGGCCCAGGAGTG(SEQ ID NO: 41) | 4626 - 5643 | yes | 68.5° | 45s | 1:30m |

**Supplementary Table B:** Sequencing primer for SNP Screening

| Primer ID | sequenze |
|---|---|
| Seq1_-3308 | CCCCTGGACTTCCATCCCTA (SEQ ID NO: 42) |
| Seq1_-2966 | TGCTGAGACTATAGGTGCGC (SEQ ID NO: 43) |
| Seq1_-2607 | AAGTGCTGGGATTACAGGAG (SEQ ID NO: 44) |
| Seq2_-2287 | CTGGGATTACAGGTGTGAGC (SEQ ID NO: 45) |
| Seq2_-1936 | CTCTAGAACCCCTTAGGTGC (SEQ ID NO: 46) |
| Seq3_-1910 | CGTGGTTACCTACCTCCTCTC (SEQ ID NO: 47) |
| Seq3_-1512 | TCAGCTCACTGCAACCTCCG (SEQ ID NO: 48) |
| Seq3_-1289 | GGCCTCCTCTCATTTTGAGC (SEQ ID NO: 49) |
| Seq4_-1267 | ATGTAAATGCCTACCGGCC (SEQ ID NO: 50) |
| Seq4_.940 | AGGGATCCTGGATCTACTCC (SEQ ID NO:51) |
| Seq5_-616 | TGTCTGTGGGTGAGCCTGTG (SEQ ID NO: 52) |
| Seq5_-222 | ATTGGTCTTCGTGTGCCGGC (SEQ ID NO: 53) |
| Seq6_-148 | TAGCTTCTTCGCCTCCGCCA (SEQ ID NO: 54) |
| Seq6_193 | GCTCACCCAGGCAGACCCCG (SEQ ID NO: 55) |
| Seq7_606 | ATGGGGTGTAGCCTCTTGAGAGG (SEQ ID NO: 56) |
| Seq7_992 | AAACTTTGTCCTGGGAGGGAGG (SEQ ID NO: 57) |
| Seq8_1301 | ACTCCTGACCAGGGTGATGC (SEQ ID NO: 58) |
| Seq8_1602 | GCCGGTCAGAGTTGAGTTTC (SEQ ID NO: 59) |
| Seq8_1876 | AGCATGGGCCCCTAACCTTG (SEQ ID NO: 60) |
| Seq9_2185 | AGTGGGAACCTCAAGGCCTC (SEQ ID NO: 61) |
| Seq9_2443 | AACAGCTGTGGCAACGCACG (SEQ ID NO: 62) |
| Seq9_2790 | ACCAACATCCACGAGCTGCG (SEQ ID NO: 63) |
| Seq10_2916 | TTCCCAGCCACTCCTCACTG (SEQ ID NO: 64) |
| Seq10_3258 | TGCCACCATGCCTGGCTAC (SEQ ID NO: 65) |
| Seq10_3616 | ACAGGTGTAAGCCACTGCGC (SEQ ID NO: 66) |
| Seq11_3935 | GGCCGCGCTGATGAACTGAGAATC (SEQ ID NO: 67) |
| Seq12_4187 | AGAGAGAGAGGAGAGGACGGTGAGC (SEQ ID NO:68) |
| Seq12_4590 | TGTCCCCAGCTTGCTGGAG (SEQ ID NO: 69) |
| Seq13_4626 | CGAGGACATCCGGTGGATGA (SEQ ID NO: 70) |
| Seq13_5643 | AAATGCCAGGCCCAGGAGTG (SEQ ID NO: 71) |

**Supplementary Table C:** Investigated polymorphisms at the *ATGL* locus.

| Number | Position [a] | Variation | Location | % Minor allele | % Heterozygosity |
|---|---|---|---|---|---|
| 1 | -2210 | T/A | Promoter | 14.6 | 25.2 |
| 2 | -2014 | G/A | Promoter | 3.8 | 7.3 |
| 3 | +550 | G/A | Intron2 | 26.3 | 37.9 |
| 4 | +2847 | C/T | Exon5 L219F | 0.02 (n=1) | - |
| 5 | +2870 | C/T | Exon5 L226L | 0.6 | 1.1 |
| 6 | +2904 | A/G | Intron5 | 41.5 | 47.9 |
| 7 | +3871 | C/G | Exon6 N252K | 1.0 | 2.0 |
| 8 | +4091 | C/G | Exon7 P291 P | 23.2 | 34.6 |
| 9 | +4710 | G/T | Exon9 L389L | 0.9 | 1.5 |
| 10 | +5071 | C/T | Exon10 P481 L | 26.0 | 37.5 |
| 11 | +5274 | TGCA/* | 3'UTR | 26.3 | 37.5 |

(continued)

| Number | Position [a] | Variation | Location | % Minor allele | % Heterozygosity |
|---|---|---|---|---|---|
| 12 | +5392 | G/A | 3'UTR | 44.2 | 49.0 |

[a] Relating to the first position of the translation starting point ATG (according to NCBI build 35, dbSNP Build 123 from 2004-11-08)

**Supplementary Table D:** Patient characteristics of 2334 participants from the three recruited groups of subjects. Values given are percentage or mean $\pm$ SD.

| | | Gastric bypass (n=1101) | Coronary heart disease (n=469) | Controls (n=864) |
|---|---|---|---|---|
| Females (%) | | 82% | 24% | 52% |
| Age < 30 yrs | | 13% | 0% | 0% |
| | 30-40 | 22% | 2% | 3% |
| | 40-50 | 30% | 21% | 38% |
| | 50-60 | 27% | 52% | 34% |
| | 60+ | 8% | 25% | 24% |
| BMI (kg/m$^2$) | | 45.8$\pm$7.6 | 28.2$\pm$5.0 | 27.6$\pm$4.9 |
| Type 2 diabetes | | 20.1% | 19.0% | 7.3% |
| Cardiovascular event | | 1.8% | 100% | 2.1 % |
| Free fatty acids (mg/dL) | | 7.9$\pm$3.0 | 8.7$\pm$5.2 | 7.0$\pm$3.7 |
| Triglycerides (mg/dL) | | 185$\pm$105 | 208$\pm$142 | 156$\pm$105 |
| Glucose (mg/dL) | | 103$\pm$35 | 101$\pm$47 | 90.7$\pm$17.7 |

It will be clear that the invention may be practiced otherwise than as particularly described in the foregoing description and examples. Numerous modifications and variations of the present invention are possible in light of the above teachings and, therefore, are within the scope of the appended claims.

**References referred to in Examples 1 to 6**

1. Higgins,M. et al. NHLBI Family Heart Study: objectives and design. Am. J. Epidemiol. 143, 1219-1228 (1996).
2. Hopkins,P.N., Wu,L.L., Hunt,S.C., & Brinton,E.A. Plasma triglycerides and type III hyperlipidemia are independently associated with premature familial coronary artery disease. J. Am. Coll. Cardiol. 45, 1003-1012 (2005).
3. Stram,D.O. et al. Choosing haplotype-tagging SNPS based on unphased genotype data using a preliminary sample of unrelated subjects with an example from the Multiethnic Cohort Study. Hum. Hered. 55, 27-36 (2003).
4. Weidinger,S. et al. Association of a STAT 6 haplotype with elevated serum IgE levels in a population based cohort of white adults. J. Med. Genet. 41, 658-663 (2004).
5. Stephens,M. & Donnelly.P. A comparison of bayesian methods for haplotype reconstruction from population genotype data. Am. J. Hum. Genet. 73, 1162-1169 (2003).

**Claims**

1. A method of diagnosing a disease or a predisposition for a disease related to the presence of a molecular variant of the adipose triglyceride lipase (ATGL) gene and wherein said disease is associated with a increase of free fatty acids comprising determining in a sample the presence of at least one nucleic acid sequence selected from the group consisting of:

(a) a nucleic acid sequence encoding adipose triglyceride lipase, wherein in the exon as indicated in column "Exon" of the following Table A the nucleotide as indicated in column "Replaced nucleotide" of Table A corresponding to the position as indicated in column "Position in wild-type" of Table A of the nucleotide sequence of the wild-type adipose triglyceride lipase as depicted in SEQ ID NO: 1 (Figures 4A to C) is replaced by a different nucleotide

Table A

| Exon | Replaced nucleotide | Position in wild-type |
|------|--------------------|----------------------|
| exon 5 | C | 870 |
| exon 9 | G | 4710 |

(b) a nucleic acid sequence comprising at least 15 nucleotides of the nucleic acid sequence of (a) and comprising one or more of the mutations as defined in (a);

(c) a nucleic acid sequence comprising a nucleotide sequence as shown in any one of SEQ ID NOs: 8 and 12 and having a mutation as defined in (a); and

(d) a nucleic acid sequence which hybridizes to a nucleotide sequence defined in any one of (a) to (c) and encoding a functional or non-functional ATCL and having a mutation as defined in (a)

2. The method of claim 1, wherein said disease is type 2 diabetes mellitus (T2DM).

3. The method of claim 1 or 2, comprising PCR, MALDI-TOF, ligase chain reaction, NASBA, restriction digestion, direct sequencing, nucleic acid amplification techniques, hybridization techniques or immunoassays.

4. A nucleic acid molecule comprising a nucleotide sequence encoding adipose triglyceride lipase and comprising the two mutations as defined in claim 1(a).

5. A diagnostic composition comprising the nucleic acid sequence as defined in claim 4 as a suitable marker for diagnosis of a disease which is associated with an increase of free fatty acid level.

**Patentansprüche**

1. Verfahren zur Diagnose einer Krankheit oder einer Veranlagung für eine Krankheit im Zusammenhang mit der Gegenwart einer molekularen Variante des adipöse Triglycerid-Lipase (ATGL)-Gens und wobei die Krankheit mit einem Anstieg der freien Fettsäuren assoziiert ist, umfassend die Bestimmung der Gegenwart von mindestens einer Nucleinsäuresequenz in einer Probe ausgewählt aus der Gruppe bestehend aus:

(a) einer Nucleinsäuresequenz, die adipöse Triglycerid-Lipase codiert, wobei in dem Exon, wie in der Spalte "Exon" der folgenden Tabelle A angegeben, das Nucleotid, wie in der Spalte "Ersetztes Nucleotid" von Tabelle A angegeben, entsprechend der Position, wie in der Spalte "Position im Wildtyp" von Tabelle A angegeben, von der Nucleotidsequenz des Wildtyps der adipösen Triglycerid-Lipase, wie in SEQ ID NO: 1 (Figuren 4A bis C) gezeigt, durch ein anderes Nucleotid ersetzt ist

| Exon | Ersetztes Nucleotid | Position im Wildtyp |
|------|--------------------|---------------------|
| Exon 5 | C | 2870 |
| Exon 9 | G | 4710 |

(b) einer Nucleinsäuresequenz umfassend mindestens 15 Nucleotide der Nucleinsäuresequenz nach (a) und umfassend eine oder mehrere der Mutationen wie in (a) definiert;

(c) einer Nucleinsäuresequenz, umfassend eine Nucleotidsequenz, wie in einer der SEQ ID NOs: 8 und 12 gezeigt, und mit einer Mutation wie in (a) definiert; und

(d) einer Nucleinsäuresequenz, die mit einer Nucleotidsequenz, wie in einem von (a) bis (c) definiert, hybridisiert und eine funktionelle oder nichtfunktionelle ATGL codiert und eine Mutation wie in (a) definiert hat.

2. Verfahren nach Anspruch 1, wobei die Krankheit Typ-2-Diabetes mellitus (T2DM) ist.

3. Verfahren nach Anspruch 1 oder 2, umfassend PCR, MALDI-TOF, Ligase-Kettenreaktion, NASBA, Restriktionsverdau, direkte Sequenzierung, Nucleinsäure-Amplifikationstechniken, Hybridisierungstechniken oder Immunassays.

**4.** Nucleinsäuremolekül, umfassend eine Nucleotidsequenz, die adipöse Triglycerid-Lipase codiert, und umfassend die zwei Mutationen wie in Anspruch 1(a) definiert.

**5.** Diagnostische Zusammensetzung, umfassend die Nucleinsäuresequenz wie in Anspruch 4 definiert als einen geeigneten Marker zur Diagnose einer Krankheit, die mit einer Zunahme des freie Fettsäurespiegels assoziiert ist.

**Revendications**

**1.** Procédé de diagnostic d'une maladie ou d'une prédisposition à une maladie liée à la présence d'un variant moléculaire du gène de l'adipose triglycéride lipase (ATGL) et dans lequel ladite maladie est associée à une augmentation des acides gras libres comprenant la détermination dans un échantillon de la présence d'au moins une séquence d'acide nucléique choisie dans le groupe constitué par :

(a) une séquence d'acide nucléique codant pour l'adipose triglycéride lipase dans laquelle, dans l'exon indiqué dans la colonne "Exon" du Tableau A suivant, le nucléotide indiqué dans la colonne "Nucléotide remplacé" du Tableau A qui correspond à la position indiquée dans la colonne "Position dans le type sauvage" du Tableau A de la séquence nucléotidique de l'adipose triglycéride lipase de type sauvage représentée dans SEQ ID No: 1 (Figures 4A à C) est remplacé par un nucléotide différent

Tableau A

| Exon | Nucléotide remplacé | Position dans le type sauvage |
|------|--------------------|------------------------------|
| Exon 5 | C | 2870 |
| Exon 9 | G | 4710 |

(b) une séquence d'acide nucléique comprenant au moins 15 nucléotides de la séquence d'acide nucléique indiquée en (a) et comprenant une ou plusieurs des mutations définies en (a) ;
(c) une séquence d'acide nucléique comprenant une séquence nucléotidique représentée dans l'une quelconque des SEQ ID Nos: 8 et 12 et comprenant une mutation telle que définie en (a) ; et
(d) une séquence d'acide nucléique qui s'hybride à une séquence nucléotidique définie dans l'un quelconque des points (a) à (c) et codant pour une ATGL fonctionnelle ou non fonctionnelle et comprenant une mutation telle que définie en (a).

**2.** Procédé selon la revendication 1, dans lequel ladite maladie est le diabète sucré de type 2 (T2DM).

**3.** Procédé selon la revendication 1 ou 2, comprenant de la PCR, du MALDI-TOF, de l'amplification en chaîne par ligase, de la NASBA, de la digestion par restriction, du séquençage direct, des techniques d'amplification d'acide nucléique, des techniques d'hybridation ou des dosages immunologiques.

**4.** Molécule d'acide nucléique comprenant une séquence nucléotidique codant pour l'adipose triglycéride lipase et comprenant les deux mutations définies dans la revendication 1(a).

**5.** Composition de diagnostic comprenant la séquence d'acide nucléique telle que définie dans la revendication 4 à titre de marqueur approprié pour le diagnostic d'une maladie qui est associée à une augmentation du taux d'acides gras libres.

## Figure 1

# Figure 2

**Figure 3**

## Figure 4A

| | | |
|---|---|---|
| -2597 | GATTACAGGAGTGAGCCACTGTACCCGGCATTTTTATTATTTATTTATTATTATTTTTGA | -2538 |
| -2537 | GACAGAGTTTCACTCTGTCGCCCAGGCTGGAGTGCAGTGCGGCGATCTGGGCTCACTGCA | -2478 |
| -2477 | AGCTCCGCCTCCCGAGTTCACACCATTCTCCTGTCTCAGCCTCCGGAGTAGCTGGGATTA | -2418 |
| -2417 | CAGGCGCCCACCACCACACCTGGCTATTTTTTTTTTTTTGTATTTTTAGTAGAGACAGGGT | -2358 |
| -2357 | TTCACCATGGTAGCCAGGATGGTCTCGATCTCCTGACCTCGTGATCCACTCACCTCAACC | -2298 |
| -2297 | TCCCAAAGTGCTGGGATTACAGGTGTGAGCCACTGTGCCTGGCCTATTTTTAAATTTTTT | -2238 |
| -2237 | GTAGGGATGGAATCAAGCTATGTTGCCTAGGCTGGTCTCAAACTTCTGGACTCAAGCAAT | -2178 |
| -2177 | CCCCCCACCTTGGCCTCCCAGAGTGCTGGGATTATAGGTGTGAACCACTGTGCCCAGCCT | -2118 |
| -2117 | AGAAGGAGTCGCTTTAAGTCAGGTATGGTCACGTCCTCCCTGCTTGACCCTCTCCCATGG | -2058 |
| -2057 | CTGGGCTTCAGCTACTCTCTCCCCTCACTCCCCGACAATCTTCGCCTGTCCTGGTCGGCT | -1998 |
| -1997 | GCCTGGGTCTGCTCATTTACCCTTCAAAATTCAACTCAGCATCACTTCCCACAGGGAAGC | -1938 |
| -1937 | CCTCCAGAACCCCTTAGGTGCCCCACACGTGGTTACCTACCTCCTCTCACTCATTTTTCT | -1878 |
| -1877 | TTTTTCTTTTTTTTTTTTTTTTTTTTGAGAAGAAGTCATGCTCTGTCACCTGGCCAGGCTGGA | -1818 |
| -1817 | GTGCAGTGGCACAATCTCAGTTCACTGCAACCTCTGCCTCCCGGGTTCAAGTGACTCTCT | -1758 |
| -1757 | TGCCTCAGCCTCCCGAGTAGCTAGGACTACATGCGTGCACTACCACACCTGGCAAATTTT | -1698 |
| -1697 | TGTATTTTCAGTAGAGACAGGGTTTTGCCATGTTGGCCTGGCTGGTCTCAAACTCCTGAC | -1638 |
| -1637 | CTCAGGTGATCTGCCCTCCTCGGCCTCCCAAAGTGCTGGGATTACAGGCATGGGCCACTG | -1578 |
| -1577 | TGCCCGGCCTCTTTTTAGGACGGAGCCTCGGCTCTGCCCCCCAGGCTGGAGTGCAGTGGTG | -1518 |
| -1517 | CGATCTCAGCTCACTGCAACCTCCGCCTCCCAGGTTCAAGTGATTCTCCTGCCTCAGCCT | -1458 |
| -1457 | CCCGAGTAGCTGGGATTACAGGCGTGTGCCACCCTGACCGGCTAATTTTTGTATTTTTGG | -1398 |
| -1397 | TAGAGACAGCGTTTCACCAGCCAGGCGGTCTCAAACTCCCGACTTTCTGTGATCCGCCCG | -1338 |
| -1337 | TCTTGGCCTCCCCAAGTGCTGGGATTACAGGTGTGAGCTTCCACACCCGGCCTCCTCTCA | -1278 |
| -1277 | TTTTGAGCTTATGTAAATGCCTACCGGCCCCCAGTAGGGCTGGGGCAGGGTTGACTTTCC | -1218 |
| -1217 | AGTGCTGTCTGGGATGGCTGGGAGCAAAGAGGCTGAGGACTGGGCAGCATCCCCCTAGAT | -1158 |
| -1157 | GGCCCCATCCTGTTCTGGCCCTGTTCTGGCTTCCCTAACTCAGCTTGGGTTGGGGGCAGC | -1098 |
| -1097 | CCCAAGCCCCATCCCAGACCCTCTGCAGCTCTGGCAACAGCTAGGTCCAGAGATGACCTC | -1038 |
| -1037 | AGCCTGGGGAGCCAGTGTTCCTGGGGAGACAGAATCTCATTGCCTCCCACCCCAACCTGA | -978 |
| -977 | GAAAGCCTCTCGTTTGGGGGTGGGGTGAGGTTCAGGGAGGGATCCTGGATCTACTCCAAG | -918 |
| -917 | CCCTGTTTGCTCCACTGACCAGGAGAAAAATGCGGAAAAGGGGTGAAAGGGTGACATCAC | -858 |
| -857 | CCAGGATTGCTCCACCTCTGGGGCAATTATCGGGAGCAGGGCGGCCCCAGTCAGACGCAG | -798 |
| -797 | GCAGCCCCAAAGCCTGAACAGGCAGGGCCAGACCCAGGTAAGAAGCCAAGGTCCGGAAGG | -738 |
| -737 | GTCGCGGGCGCGGGGCAGGGCTGAGGCCCCACGTCAGGTCGCGTGGGTCTGCGGAGCTGG | -678 |
| -677 | GGTCCCCCACGAAGGGTTTGCGGGACTCGCATCCGGCCTTGGCCTTGGATGTTTATGGGT | -618 |
| -617 | CTGTCTGTGGGTGAGCCTGTGGGTCCCGGGGCTCCCGAGGCCTGTGCGGCTTTCAGGTCT | -558 |
| -557 | GGGGGTGCTGCCGGTGCGCCTCTGTCCAGGGACCAGCTCCTCTGTCCCGGGTGGGTGTGG | -498 |
| -497 | GAGGCGGACCCGGTGGGGAGGGGCGGTGGCTGCCCTGAAACCAGAGCTCCCTCTCGCCGG | -438 |
| -437 | TACCCAGGTCTGTGTGAGGCTTCCGGCCCCACGGCTGCGGCCTGCGCCCAGCCCACCCTA | -378 |
| -377 | CACCCCTAGGCGTGTGCCCCCAGCCGTGCCCAGCAGGGCGGGAGGGAGGGCAGAAGCTTT | -318 |
| -317 | CTCCGGGCGGCAGCGGAGGGCGGGGCTCCAGCGCGGGGGGGCCGGGTCAAGGACGGGGGCG | -258 |
| -257 | GGTCCCGAGGGCACGGCCGTTCCCTGCGCGCCCCGATTGGTCTTCGTGTGCCGGCCCCGC | -198 |
| -197 | CCCCGCCGTGAGTCCCACACTTAAAAGCGCCGCCTCCGCGCCGCCCGCGTAGCTTCTTCG | -138 |
| -137 | CCTCCGCCAGCGGGGACCCCGAGCTAGAGCCGCAGCGGGACCTGCCCGGCCCCCGGCTCC | -78 |
| -77 | AGCGAGCGAGCGGCGAGCAGGCGGCTCACAGAGGCCTGGCCGCCCACGGAACCCGGGGCC | -18 |
| -17 | CGGCGGCCGCCGCCGCGATGTTTCCCCGCGAGAAGACGTGGAACATCTCGTTCGCGGGCT | 43 |
| 44 | GCGGCTTCCTCGGCGTCTACTACGTCGGCGTGGCCTCCTGCCTCCGCGAGCACGCGCCCT | 103 |
| 104 | TCCTGGTCGCCAACGCCACGCACCATCTACGGCGCCTCCGCCCGGGGCGCCTCACGGCCCACGG | 163 |
| 164 | CGCTGGTCACCGGGGTCTGCCTGGGTGAGCGGGGCCGGGGCGGCAGGCGGAGGGGCTGGC | 223 |
| 224 | GGGAAGGCCGTGCGGGGCCGGGGGATGGTCTCCGTGAGCGGAGGGGCCCCGCGGCCGAGTC | 283 |
| 284 | GGTGGGTACCGTGGGGAGGGTTCCGGTCCGCGCCTGGGGAACCCGGCGAGGATCCAATCC | 343 |
| 344 | GGGTCGCTGGCCTGGGGGCGGGGCCTGGTTTGTTTTGCTCCGAGGGTGCCCGGGGCCCGC | 403 |
| 404 | CTGTCTGGCGCAAAGGTTTGGGGGCGGGCAGGTGCAGCAAAAGCTGCTTAATTGGACAGA | 463 |
| 464 | AAAAGTAACTCGTGGGCGGGACCGTGAGAATCCGAGGAGGGGACCTAAGTCACCCCGTGGC | 523 |
| 524 | CCTGGATATTACAACCGGTGCCAGCGGTCGGTCACAGTGTCCTGGGCTTCCGCAGTTGCA | 583 |
| 584 | GGCTGCTATCAGGGGGCCTGGCATGGGGGTAGCCTCTTGAGAGGCGTCTGGGAACCGTGA | 643 |
| 644 | CCTGGTCCTTCCCAAGGGCCCGGAGGAGGAGCCCCCGCTGAGGTCAGGGCTGGGCGCGAAG | 703 |
| 704 | GGGCTCTCCTGCCCTCTCCTTGCACACGGTGCCCTGTGGCCTGGCTCCCCGCTGCGGCCC | 763 |
| 764 | ACCGCGTTTGCACACTTCATGGGTGAGGGTGCTTCTGGGCTCTGGTGCCTGGGTCAGGAG | 823 |
| 824 | TGGATGGGTCTCTGTGTGCTGGGCTGGCCTCGGCTCGCACCATCGGCTGCCATGAGGGAG | 883 |
| 884 | TGATGTTTACAGCACACGACTTCAGGAGCCTGTGAGGACACCCAAGATGACAGGGGCACT | 943 |
| 944 | CTGCTCAGCAGGAGCCTGTCCGGGGCTCACCCCTGCCCTCTTCCTCTGAACTTTGTCCTG | 1003 |
| 1004 | GGAGGGAGGGGGCTGGACCACAGAAGTGAACCTCTCAGGTCCCAATAACTAGAGCTATTA | 1063 |
| 1064 | TTGGGAACAGCCCCCTCCAGCCCCCTCCCTCTCATGGAGGCCTTCAGACAAGCGCCTCAG | 1123 |
| 1124 | TCGGCCCCCTGGCTTCAAGATACATCAAGGTCCCTGCTACCCGAGCCAAGGACACCTCTC | 1183 |
| 1184 | CCACAGTGCTTCCCCACCCTGCTCCCAACAAGACTCCTTCTCACGGGTGGTCTTGCACAG | 1243 |

## Figure 4B

```
1244  CTGGAGCCCATAGTGCAGCAGGTGCTGGCTGAAGAGCCCCTGGCTCCACACTGCCCCACT  1303
1304  CCTGACCAGGTGATGCACTGGAGGAGGGCTTGGACCTCAGCTCCTCCCTCAGTGCTCCC   1363
1364  GACCACTTCCAGGGACTATCCCCAAGCTGGCCAGCACTCCTGCGCCCCAAGAGGAGTGTT  1423
1424  TGGGTGCTTCCTCTTGGCTGCAGTGGGACACAGGTGTGCCTTCCTAGGAACTGGGCCCT   1483
1484  GACTACTTCCAGCCCAACACTCCCGGGCCTGTGAACTGTGACCTGTGTGCCGGGATGGGT  1543
1544  TTTGTGGGTCTGCCCCATCCCCGCACTGCTGGATCTGGCCAAGTGGGTGAAGGCTAAGGC  1603
1604  CGGTCAGAGTTGAGTTTCTGCCTTGTCCCCTCTCCTGGGCTAGATGCCACACCAGGCCCA  1663
1664  GTGACTCATAGGGCAGGCAGTTGGGAAATACCAGGCAGAGGGCAGGTCCTGGTCTCAGCT  1723
1724  GGCCAGCCTCTGCTGTCTGCCATCCCAGGGGAGGTGGCCAAAGTCCCAACTGTGAGCCAG  1783
1784  GCCCCACATTCACTGGGCCTCCTCCAGGGTCTGTATGCCATGGAACCCTGGACATGGGGC  1843
1844  TATGAAGGAAGGTGGGTGTTGCTAAGCCCAGGAGCATGGGCCCCTAACCTTGGCCCTGTG  1903
1904  CCCCAGGTGAGGCTGGTGCCAAGTTCATTGAGGTATCTAAAGAGGCCCGGAAGCGGTTCC  1963
1964  TGGGCCCCCTGCACCCCTCCTTCAACCTGGTAAAGATCATCCGCAGTTTCCTGCTGAAGG  2023
2024  TCCTGCCTGCTGATAGCCATGAGCATGCCAGTGGGCGCCTGGGCATCTCCCTGACCCGCG  2083
2084  TGTCAGACGGCCGAGAATGTCATTATATCCCACTTCAACTCCAAGGACGAGCTCATCCAGG  2143
2144  TGGGGCCTGGTGGAGCCATGCTGGGTGGCCGGTGGGGGGGGCAGTGGGAACCTCAAGGCCT  2203
2204  CTGCTCATTCTCTCCCACTCTGTCCCTGCCCTGAAGGCCAATGTCTGCAGCGGTTTCATC  2263
2264  CCCGTGTACTGTGGGCTCATCCCTCCCTCCCTCCAGGGGGTGGTGAGTATTCCTAGCCCT  2323
2324  GGACACCTTCTATGGGGTGGGCCGTGGGATGAGGGACAGAGGAGGAGGCCGCCTAGAGCC  2383
2384  ATCCTTCAGGCCCTTGCTCTGCCACCGCCTGTTACCCACTTCCCCTGTGTTACTCAAGAA  2443
2444  ACAGCTGTGGCAACGCACGCTTCCTGGCCCCCCATCCCTTCCTCCGTCCCTGCCCTCCCC  2503
2504  CGTCTACCATCTGCTCAGTGCCCAGGCTGGCCCACAGCCAGTGCCCAGTGGGTAAAACGC  2563
2564  TCAAATGAGGTAGCCACTGAATGGGGCCCTTGGTGGCCGGGTGGGGTGGCTGGGGTGGGT  2623
2624  GGCCAGTGCAGCCACAGGCCCTCACATACGGTCCTGTCTGTGTGTCCCGTGGAAGCGCTA  2683
2684  CGTGGATGGTGGCATTTCAGACAACCTGCCACTCTATGAGCTTAAGAACACCATCACAGT  2743
2744  GTCCCCCTTCTCGGGCGAGAGTGACATCTGTCCGCAGGACAGCTCCACCAACATCCACGA  2803
2804  GCTGCGGGTCACCAACACCAGCATCCAGTTCAACCTGCCAACCTCTACCGCCTCTCCAA   2863
2864  GGCCCTCTTCCCGCCGGAGCCCCTGGTGAGCTCTGCTCCGAGGACTGTGGCCTTCCCAGC  2923
2924  CACTCCTCACTGGGCACCAAGGGAGAACACTGATCCTTTGACTTCTGAGTGCCCAGGGTA  2983
2984  GGGTGGTGGGAGCCTCTTCTGAGGGCTGGGGAAAGCGCACAACCTTTCTAGGGGCAGATG  3043
3044  GCCCATCCAACCTCTCTGTCTAGCTGCTCTGTCCAGGCTCCCTGTCCAGTCTCTCTCTCT  3103
3104  TTTTTTTTTTTTTTTTTTTGTTTGAGACGGAGTCTCGCTCTGTTGCCAAGGCTGGAGTGCAG  3163
3164  TGGCAGGATCTCAGCTCACTGCAACCTCTGCCTCCAAGGTTCAAGCTATTCTCCTGCCTC  3223
3224  AGCCTCCCCATTAGCTGGGATTACAGGCGCCTGCCACCATGCCTGGCTACTTTTTGTATT  3283
3284  TTTAGTAAAGAAATAGGGTTTCACCATGTTGGCCAGGCTGGCCTCAAACTATTTTATTTT  3343
3344  TTTATTTTTTTTTTGAGATGAAGTCTCACACTGTCACCCAGGCTGGAGTGCAGTGGCG   3403
3404  ATCTCCTCTCACTGCAAGCTCCACCTCCCGGGTTCCTGCCATTCTCCTGCCTCAGCCTCC  3463
3464  CCAGTAGCTGGGACTACAGGTGCCCACCACCACGCCTGGCTAATTTTTTGTATTTTTAGT  3523
3524  AGAGACGGGGTTTCACCGTGTTAGCCAGGATGGTCTTGATCTCCTGACCTCGTCATCCGC  3583
3584  CTGCCTCAGCCTCCCAAAGTGCTGGGGTTACAGGTGTAAGCCACTGCGCCCGGCTTTCTG  3643
3644  TCCAACCTCCCTGTACAGCTTTGATCTTGTTCTGGATCTAGGATAGGTTTTGGGGTTGGG  3703
3704  AGCTGCGGGTAGTGAAGGGAGGTGGCTGTTGGGGGTGCCAGGGATTCCAGGGGCAGAACG  3763
3764  GGCATCCCTGGCTCCCTCCGCTCCATTTAACCCTCCTCACTGCAGGTGCTGCGAGAGATG  3823
3824  TGCAAGCAGGGATACCGGGATGGCCTGCGCTTTCTGCAGCGGAACGGTGCGCGGACCCGG  3883
3884  GCGGGAGAGGGCGGGGTGGGCTCGGCTCTGCTACCCCCTGCGGGCCGCCGGCCGGCTGCAT  3943
3944  GAACTGAGAATCCCTTCTCTCCCCAACCCCAGGCCTCCTGAACCGGCCCAACCCCTTGCT  4003
4004  GGCGTTGCCCCCCGCCCGCCCCCACGGCCCAGAGGACAAGGACCAGGCAGTGGAGAGCGC  4063
4064  CCAAGCGGAGGATTACTCGCAGCTGCCCCGGAGAAGATCACATCCTGGAGCACCTGCCCGC  4123
4124  CCGGCTCAATGAGGGTGCGCACCTGGGGGACGGGGGAGGAGGGGAGGCAGGAGGGAA    4183
4184  AGAGAGAGAGGAGAGGACGGTGAGCAGGGGAGGGAAGCCGAGCGGGTCCTGGGCCCCGCT  4243
4244  GCCTCCACTGGCCGCCGACCTCCCGCCCACCCGCAGCCCTGCTGGAGGCCTGCGTGGAGC  4303
4304  CCACGGACCTGCTGACCACCCTCTCCAACATGCTGCCTGTGCGTCTGGCCACGGCCATGA  4363
4364  TGGTGCCCTACACGCTGCCGCTGGAGAGCGCTCTGTCCTTCACCATCCGGTGTGAGGGCT  4423
4424  GGGGGGTCGGGAGAGGGGCGGGGTGGGCTCAGGGACGGACTTTGGGATCATCCGCGAGTGATGGTTAC  4483
4484  CAGGGAAGGTGGGGTGGGGTGAGCAGTGCCAAGTCAGGGCACAGACAGGGCCCGGCGGGT  4543
4544  GGGCATGTGGGTCTGGCCAAGTCCCTGAACGCGCCGCTCGCCCTGTCCCCAGCTTGCTGG  4603
4604  AGTGGCTGCCCGACGTTCCCGAGGACATCCGGTGGATGAAGGAGCAGACGGGCAGCATCT  4663
4664  GCCAGTACCTGGTGATGCGCGCCAAGAGGAAGCTGGGCAGGCACCTGCCCTCCAGGTGAG  4723
4724  CCGCCGACCGCGCGTCCACCCGGGGCCCGCGGTGCTAGCGCCGGGAGCTGAAGCCCTCCC  4783
4784  TGCCGCATCCCTGCCCCGCAGGCTGCCCGAGCAGGTGGAGCTGCGCCCGCGTCCAGTCGCT  4843
4844  CCCGTCCGTGCCGCTGTCCTGCGCCGCCTACAGAGAGGCACTGCCCGGCTGGATGCGCAA  4903
4904  CAACCTCTCGCTGGGGGACGCGCTGGCCAAGTGGGAGGAGTGCCAGCGCCAGCTGCTGCT  4963
```

# Figure 4C

```
4964    CGGCCTCTTCTGCACCAACGTGGCCTTCCCGCCCGAAGCTCTGCGCATGCGCGCACCCGC    5023
5024    CGACCCGGCTCCCGCCCCCGCGGACCCAGCATCCCCGCAGCACCAGCCGGCCGGGCCTGC    5083
5084    CCCCTTGCTGAGCACCCCTGCTCCCGAGGCCCGGCCCGTGATCGGGGCCCTGGGGCTGTG    5143
5144    AGACCCCGACCCTCTCGAGGAACCCTGCCTGAGACGCCTCCATTACCACTGCGCAGTGAG    5203
5204    ATGAGGGGACTCACAGTTGCCAAGAGGGGTCTTTGCCGTGGGCCCCCTCGCCAGCCACTC    5263
5264    ACCAGCTGCATGCACTGAGAGGGGAGGTTTCCACACCCCTCCCCTGGGCCGCTGAGGCCC    5323
5324    CGCGCACCTGTGCCTTAATCTTCCCTCCCCTGTGCTGCCCGAGCACCTCCCCCGCCCCTT    5383
5384    TACTCCTGGGAACTTTGCAGCTGCCCTTCCCTCCCCGTTTTTCATGGCCTGCTGAAATAT    5443
5444    GTGTGTGAAGAATTATTTATTTTCGCCAAAGCACATGTAATAAATGCTGCAGCCCAGCCT .  5503
5504    CTGCCCACTTTGTGTGTATGTGACCGCCTGCTTACTTGCAGTGAGAGCCTGGTGGCCAGG    5563
5564    GTCTGGCCCTACCTTGGCTGACCAGCCTCTCCACAGCTGCAGGCCAGGTCTCCCAGCGTC    5623
5624    GCACTCCTGGGCCTGGCATTTGGAACCTGCCAGGCTGGCCTGGGAACACCCCCCTACAGG    5683
5684    CACATATGAACGTACTGCATTCCTGCCGACCCCCCTGTCTAGGATGCATCCACACCCCCC .  5743
5744    CCAATTTTGCCCAGCAGCCTCCTGGCTGACCCTTGGCCACAGCCTTCTGAGGGCCAATGG    5803
5804    AAATATTTGGGACCAAGATTCTTGGTAAATAAAAACGAAAATGTTTGCAAAAGGTGTCGC    5863
5864    CTGCCCCTCCCCTCACACGGTGACACAGTGGGGTGTGTGACTGTTTTGGGGCTTGGCCAG    5923
5924    CTGTGGGGGCTGAGGTTCCTGAAACCAAGCACAGGGGACCTGGGAAAGCTGGGCCCCTGC    5983
5984        TCCACTTGGGCGTTT        6043    (SEQ ID NO: 1)
```

This sequence describes the Insertion type for SNP11 which is considered the wildtype

29

# Figure 5

```
   1 GCGGCCCCAGTCAGACGCAGGCAGCCCCAAAGCCTGAACAGGCAGGGCCAGACCCAGCTT
  61 CTTCGCCTCCGCCAGCGGGGACCCCGAGCTAGAGCCGCAGCGGGACCTGCCCGGCCCCCG
 121 GCTCCAGCGAGCGAGCGGCGAGCAGGCGGCTCACAGAGGCCTGGCCGCCCACGGAACCCG
 181 GGGCCCGGCGGCCGCCGCCGCGATGTTTCCCCGCGAGAAGACGTGGAACATCTCGTTCGC
 241 GGGCTGCGGCTTCCTCGGCGTCTACTACGTCGGCGTGGCCTCCTGCCTCCGCGAGCACGC
 301 GCCCTTCCTGGTGGCCAACGCCACGCACATCTACGGCGCCTCGGCCGGGGCGCTCACGGC
 361 CACGGCGCTGGTCACCGGGGTCTGCCTGGGTGAGGCTGGTGCCAAGTTCATTGAGGTATC
 421 TAAAGAGGCCCGGAAGCGGTTCCTGGGCCCCCTGCACCCCTCCTTCAACCTGGTAAAGAT
 481 CATCCGCAGTTTCCTGCTGAAGGTCCTGCCTGCTGATAGCCATGAGCATGCCAGTGGGCG
 541 CCTGGGCATCTCCCTGACCCGCGTGTCAGACGGCGAGAATGTCATTATATCCCACTTCAA
 601 CTCCAAGGACGAGCTCATCCAGGCCAATGTCTGCAGCGGTTTCATCCCCGTGTACTGTGG
 661 GCTCATCCCTCCCTCCCTCCAGGGGGTGCGCTACGTGGATGGTGGCATTTCAGACAACCT
 721 GCCACTCTATGAGCTTAAGAACACCATCACAGTGTCCCCCTTCTCGGGCGAGAGTGACAT
 781 CTGTCCGCAGGACAGCTCCACCAACATCCACGAGCTGCGGGTCACCAACACCAGCATCCA
 841 GTTCAACCTGCGCAACCTCTACCGCCTCTCCAAGGCCCTCTTCCCGCCGGAGCCCCTGGT
 901 GCTGCGAGAGATGTGCAAGCAGGGATACCGGGATGGCCTGCGCTTTCTGCAGCGGAACGG
 961 CCTCCTGAACCGGCCCAACCCCTTGCTGGCGTTGCCCCCCGCCCGCCCCCACGGCCCAGA
1021 GGACAAGGACCAGGCAGTGGAGAGCGCCCAAGCGGAGGATTACTCGCAGCTGCCCGGAGA
1081 AGATCACATCCTGGAGCACCTGCCCGCCCGGCTCAATGAGGCCCTGCTGGAGGCCTGCGT
1141 GGAGCCCACGGACCTGCTGACCACCCTCTCCAACATGCTGCCTGTGCGTCTGGCCACGGC
1201 CATGATGGTGCCCCTACACGCTGCCGCTGGAGAGCGCTCTGTCCTTCACCATCCGCTTGCT
1261 GGAGTGGCTGCCCGACGTTCCCGAGGACATCCGGTGGATGAAGGAGCAGACGGGCAGCAT
1321 CTGCCAGTACCTGGTGATGCGCGCCAAGAGGAAGCTGGGCAGGCACCTGCCCTCCAGGCT
1381 GCCGGAGCAGGTGGAGCTGCGCCGCGTCCAGTCGCTGCCGTCCGTGCCGCTGTCCTGCGC
1441 CGCCTACAGAGAGGCACTGCCCGGCTGGATGCGCAACAACCTCTCGCTGGGGGACGCGCT
1501 GGCCAAGTGGGAGGAGTGCCAGCGCCAGCTGCTGCTCGGCCTCTTCTGCACCAACGTGGC
1561 CTTCCCGCCCGAAGCTCTGCGCATGCGCGCACCCGCCGACCCGGCTCCCGCCCCCGCGGA
1621 CCCAGCATCCCCGCAGCACCAGCCGGCCGGGCCTGCCCCCTTGCTGAGCACCCCTGCTCC
1681 CGAGGCCCGGCCCGTGATCGGGGCCCTGGGGCTGTGAGACCCCGACCCTCTCGAGGAACC
1741 CTGCCTGAGACGCCTCCATTACCACTGCGCAGTGAGATGAGGGGACTCACAGTTGCCAAG
1801 AGGGGTCTTTGCCGTGGGCCCCCTCGCCAGCCACTCACCAGCTGCATGCACTGAGAGGGG
1861 AGGTTTCCACACCCCTCCCCTGGGCCGCTGAGGCCCCGCGCACCTGTGCCTTAATCTTCC
1921 CTCCCCTGTGCTGCCCGAGCACCTCCCCCGCCCCTTTACTCCTGAGAACTTTGCAGCTGC
1981 CCTTCCCTCCCCGTTTTTCATGGCCTGCTGAAATATGTGTGTGAAGAATTATTTATTTTC
2041 GCCAAAGCACATGTAATAAATGCTGCAGCCC          (SEQ ID NO: 2)
```

30

# Figure 6

```
  1 MFPREKTWNISFAGCGFLGVYYVGVASCLREHAPFLVANATHIYGASAGALTATALVTGV
 61 CLGEAGAKFIEVSKEARKRFLGPLHPSFNLVKIIRSFLLKVLPADSHEHASGRLGISLTR
121 VSDGENVIISHFNSKDELIQANVCSGFIPVYCGLIPPSLQGVRYVDGGISDNLPLYELKN
181 TITVSPFSGESDICPQDSSTNIHELRVTNTSIQFNLRN▉YRLSKA▉FPPEPLVLREMCKQ
241 GYRDGLRFLQR▉GLLNRPNPLLALPPARPHGPEDKDQAVESAQAEDYSQL▉GEDHILEHL
301 PARLNEALLEACVEPTDLLTTLSNMLPVRLATAMMVPYTLPLESALSFTIRLLEWLPDVP
361 EDIRWMKEQTGSICQYLVMRAKRKLGRH▉PSRLPEQVELRRVQSLPSVPLSCAAYREALP
421 GWMRNNLSLGDALAKWEECQRQLLLGLFCTNVAFPPEALRMRAPADPAPAPADPASPQHQ
481 ▉AGPAPLLSTPAPEARPVIGALGLQFNLRNLYRLSKALFPPEPLVLREMCKQ
```

(SEQ ID NO: 3)

# ·Figure 7A

**Sequences describing the SNP regions:**

**The upper strand is the forward strand (sense strand)**
**The lower strand is the reverse strand (complementary strand)**

The protein sequence changes, caused by a SNP variation in exons, are given below the nucleotide sequence

**SEQ ID No:4        SNP 1 (-2210)**

```
5'-ATCAAGCTATGTTGCC[T/A]AGGCTGGTCTCAAACTTCTGG-3'
3'-TAGTTCGATACAACGG[A/T]TCCGACCAGAGTTTGAAGACC-5'
```

**SEQ ID No:5        SNP 2 (-2014)**

```
5'-CTCCCCGACAATCTTC[G/A]CCTGTCCTGGTCGGCTGCCTGG-3'
3'-GAGGGGCTGTTAGAAG[C/T]GGACAGGACCAGCCGACGGACC-5'
```

**SEQ ID No:6        SNP 3, rs7925131 (+550)**

```
5'-TATTACAACCGGTGCCAGCG[G/A]TCGGTCACAGTGTCCTGGGC-3'
3'-ATAATGTTGGCCACGGTCGC[C/T]AGCCAGTGTCACAGGACCCG-5'
```

**SEQ ID No:7        SNP 4 (+2847)**

```
5'-CCAGTTCAACCTGCGCAAC[C/T]TCTACCGCCTCTCCAAGGC-3'
3'-GGTCAAGTTGGACGCGTTG[G/A]AGATGGCGGAGAGGTTCCG-5'
```

VTNTSIQFNLRN█YRLSKALFPPEPL  (NON SYNONYMOUS CODING L219F)

**SEQ ID No:8        SNP 5, rs10902224 (+2870)**

```
5'-GCCTCTCCAAGGCCCT[C/T]TTCCCGCCGGAGCCCCTGGTGA-3'
3'-CGGAGAGGTTCCGGGA[G/A]AAGGGCGGCCTCGGGGACCACT-5'
```

QFNLRNLYRLSKA█FPPEPLVLREMCKQ  (SYNONYMOUS CODING L226L)

**SEQ ID No:9        SNP 6, rs7942159 (+2904)**

```
5'-TGGTGAGCTCTGCTCCG[A/G]GGACTGTGGCCTTCCCAGCCAC-3'
3'-ACCACTCGAGACGAGGC[T/C]CCTGACACCGGAAGGGTCGGTG-5'
```

# Figure 7B

**SEQ ID No:10        SNP 7 (+3871)**

5'-CCTGCGCTTTCTGCAGCGGAA(C/G)GGTGCGCGGACCCGGGCGG-3'
3'-GGACGCGAAAGACGTCGCCTT(G/C)CCACGCGCCTGGGCCCGCC-5'

GYRDGLRFLQR█GLLNRPNPLLALP (NON SYNONYMOUS CODING N252K)

**SEQ ID No:11        SNP 8, rs1135628 (+4091)**

5'-GAGGATTACTCGCAGCTGCC(C/G)GGAGAAGATCACATCCTG-3'
3'-CTCCTAATGAGCGTCGACGG(G/C)CCTCTTCTAGTGTAGGAC-5'

SAQAEDYSQL█GEDHILEHLPARLN (SYNONYMOUS CODING P291P)

**SEQ ID No:12        SNP 9, rs11554663 (+4710)**

5'-GGAAGCTGGGCAGGCACCT(G/T)CCCTCCAGGTGAGCCGCCGA-3'
3'-CCTTCGACCCGTCCGTGGA(C/A)GGGAGGTCCACTCGGCGGCT-5'

LVMRAKRKLGRH█PSRLPEQVELR (SYNONYMOUS CODING L389L)

**SEQ ID No:13        SNP 10, rs1138693 (+5071)**

5'-AGCATCCCCGCAGCACCAGC(C/T)GGCCGGGCCTGCCCCCTTG-3'
3'-TCGTAGGGGCGTCGTGGTCG(G/A)CCGGCCCGGACGGGGGAAC-5'

PAPAPADPASPQHQ█AGPAPLLSTPAPEA (NON SYNONYMOUS CODING P481L)

**SEQ ID No:14        SNP 11 (+5274)**

5'-AGCCACTCACCAGCTGCA(*/TGCA)CTGAGAGGGGAGGTTTCCA-3'
3'-TCGGTGAGTGGTCGACGT(*/ACGT)GACTCTCCCCTCCAAAGGT-5'

**SEQ ID No:15        SNP 12, rs1138714 (+5392)**

5'-CCGCCCCTTTACTCCTG(G/A)GAACTTTGCAGCTGCCCT-3'
3'-GGCGGGGAAATGAGGAC(C/T)CTTGAAACGTCGACGGGA-5'

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SCHAFFER.** *Current Opinion in Lipidology,* 2003, vol. 14, 281-287
- **ZIMMERMANN,R. et al.** *Science,* 2004, vol. 306, 1383-1386
- **JENKINS,C.M. et al.** *J. Biol. Chem.,* 2004, vol. 279, 48968-48975
- *Methods Mol Biol.,* 1999, vol. 109, 109-21
- **SAMBROOK et al.** Molecular Cloning: A Laboratory Manual. Cold Spring Harbor Laboratory, 2001
- **RODAK.** Haematology: Clinical Principles & Applications. WB Saunders Co, 2002
- **BRUNZEL.** Fundamentals of Urine and Body Fluids Analysis. WB Saunders Co, 1994
- Cerebrospinal Fluid. Kluwer Academic Pub, 1989
- Protocols for Oligonucleotides and Analogs: Synthesis and Properties. Methods in Molecular Biology. Humana Press, 1993, vol. 20
- PCR Applications: Protocols for Functional Genomics. Academic Press, 1999
- PCR Cloning Protocols: From Molecular Cloning to Genetic. Humana Press, 2002
- Automated DNA Sequencing and Analysis. Academic Press, 1994
- **ALPHEY.** DNA Sequencing: From Experimental Methods to Bioinformatics. Springer Verlag Publishing, 1997
- **NOLLAU et al.** *Clin. Chem.,* 1997, vol. 43, 1114-1128
- Polymorphism Detection & Analysis Techniques. Eaton Pub Co, 2000
- Mutation Detection: A Practical Approach. Irl Press, 1998
- Laboratory Methods for the Detection of Mutations and Polymorphisms in DNA. CRC Press, 1997
- **ALTSCHUL.** *Nucl. Acids Res,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **THOMPSON.** *Nucl. Acids Res.,* 1994, vol. 2, 4673-4680
- **BRUTLAG.** *Comp. App. Biosci.,* 1990, vol. 6, 237-245
- **FISHMAN et al.** *J. Clin. Invest.,* 1998, vol. 102, 1369-1376
- **CHAPPELL et al.** *Oncogene,* 2000, vol. 19, 4437-4440
- **MANN et al.** *J. Clin. Invest,* 2001, vol. 107, 899-907
- **KEEN et al.** *Bone,* 1998, vol. 23, 367-371
- **CACERES ; KORNBLIHTT.** *Trends Genet.,* 2002, vol. 4, 186-93
- **SAMBROOK, RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press Oxford, 1985
- *Altschul Nucl. Acids Res.,* 1977, vol. 25, 3389-3402
- **HENIKOFF.** *Proc. Natl. Acad. Sci., USA,* 1989, vol. 89, 10915
- **TIJSSEN.** Practice and theory of enzyme immunoassays. 1985, vol. 15
- **DAVIS LG ; DIBMER MD.** Basic methods in molecular biology. Battey Elsevier, 1990
- Immunochemical methods in cell and molecular biology. Academic Press, 1987
- Methods in Enzymology. Academic Press, Inc
- *Science,* 2004, vol. 306, 1383
- **BODEN,G. ; SHULMAN,G.I.** *Eur. J. Clin. Invest,* 2002, vol. 32 (3), 14-23
- **ZECHNER,R. ; STRAUSS,J.G. ; HAEMMERLE,G. ; LASS,A. ; ZIMMERMANN,R.** *Curr. Opin. Lipidol.,* 2005, vol. 16, 333-340
- **VILLENA,J.A. ; ROY,S. ; SARKADI-NAGY,E. ; KIM,K.H. ; SUL,H.S.** *J. Biol. Chem.,* 2004, vol. 279, 47066-47075
- **NYHOLT,D.R.** *Am. J. Hum. Genet.,* 2004, vol. 74, 765-769
- **MIRANDA,P.J. ; DEFRONZO,R.A. ; CALIFF,R.M. ; GUYTON,J.R.** *Am. Heart J.,* 2005, vol. 149, 33-45
- **LAM,T.K. et al.** *Am. J. Physiol Endocrinol. Metab,* 2003, vol. 284, E863-E873
- **HIGGINS,M. et al.** NHLBI Family Heart Study: objectives and design. *Am. J. Epidemiol.,* 1996, vol. 143, 1219-1228

- **HOPKINS,P.N. ; WU,L.L. ; HUNT,S.C. ; BRINTON,E.A.** Plasma triglycerides and type III hyperlipidemia are independently associated with premature familial coronary artery disease. *J. Am. Coll. Cardiol.,* 2005, vol. 45, 1003-1012
- **STRAM,D.O. et al.** Choosing haplotype-tagging SNPS based on unphased genotype data using a preliminary sample of unrelated subjects with an example from the Multiethnic Cohort Study. *Hum. Hered.,* 2003, vol. 55, 27-36
- **WEIDINGER,S. et al.** Association of a STAT 6 haplotype with elevated serum IgE levels in a population based cohort of white adults. *J. Med. Genet.,* 2004, vol. 41, 658-663
- **STEPHENS,M. ; DONNELLY.P.** A comparison of bayesian methods for haplotype reconstruction from population genotype data. *Am. J. Hum. Genet.,* 2003, vol. 73, 1162-1169